# EUROPEAN PATENT APPLICATION

(11) **EP 4 306 172 A1**
(43) Date of publication of application: **17.01.2024**
(21) Application number: 22766319.2
(22) Date of filing: 09.03.2022
(51) Int. Cl.: A61P 37/00, A61P 29/00, A61P 17/06, A61K 31/5383

(54) **USE OF TRICYCLIC HETEROARYL-CONTAINING COMPOUND**

(30) Priority: 09.03.2021 CN 202110255275; 29.07.2021 CN 202110861899
(71) Applicant: CSPC Zhongqi Pharmaceutical Technology (Shijiazhuang) Co., Ltd., Shijiazhuang, Hebei 050035 (CN); Hangzhou Innogate Pharma Co., Ltd., Hangzhou, Zhejiang 311121 (CN); CSPC OUYI Pharmaceutical co., Ltd., Shijiazhuang, Hebei 052165 (CN)
(72) Inventor: YANG, Hanyu, Shijiazhuang, Hebei 050035 (CN); ZHANG, Hancheng, Hangzhou, Zhejiang 311121 (CN); LIU, Xibao, Shijiazhuang, Hebei 050035 (CN); CAI, Congcong, Hangzhou, Zhejiang 311121 (CN); QIN, Ning, Hangzhou, Zhejiang 311121 (CN); DAN, Mo, Shijiazhuang, Hebei 050035 (CN); LYU, Lu, Shijiazhuang, Hebei 050035 (CN); ZHANG, Dandan, Shijiazhuang, Hebei 050035 (CN); LIU, Jieru, Shijiazhuang, Hebei 050035 (CN)
(74) Representative: Newcombe, Christopher David
(86) International application number: PCT/CN2022/079887
(87) International publication number: WO 2022/188796

(57) **Abstract**

The present application provides use of Compound (I), an optical isomer thereof or a pharmaceutically acceptable salt thereof in manufacture of a medicament useful in treating a disease related to high expression or abnormal activation of JAK and SYK kinases, comprising an autoimmune disease, such as an immune-mediated skin disease, in particular psoriasis, atopic dermatitis and SLE. Compound (I), an optical isomer thereof or a pharmaceutically acceptable salt thereof according to the present invention can improve the skin lesions in mice with psoriasis, atopic dermatitis and SLE, alleviate the injury of kidney, inhibit the expansion of immune organs, reduce the level of inflammation, inhibit the increase of SLE related antibodies and cytokines in serum, and has a certain safe therapeutic window, which has a good clinical application prospect.

## Description

### Technical Field

The present invention belongs to the field of medicine, in particular to the use of a compound containing a tricyclic heteroaryl group in the preparation of a medicament for treating a disease related to high expression or abnormal activation of JAK and SYK kinases.

### Background Art

JAK (namely Janus kinase) is a class of non-transmembrane non-receptor tyrosine kinases, including four subtypes: JAK1, JAK2, JAK3 and TYK2 (tyrosine kinase 2). JAK1, JAK2, and TYK2 are widely present in various tissues and cells. JAK1 participates in mediating the inflammatory signaling pathways such as IL-6 and IFN. JAK2 can independently mediate cytokine signaling pathways such as IL-3, IL-5 and EPO. JAK3 is only present in bone marrow and lymphatic system, and mediates the signaling of IL-2, IL-4, IL-7, IL-9, IL-15 and IL-21. TYK2 participates in IFN-alpha, IL-6, IL-10 and IL-12 signaling. JAK inhibitors block the cascade amplification of the above cytokines, and participate in immune regulation and other processes by specifically inhibiting the JAK-STAT (signal transducers and activators of transcription) signaling pathway.

SYK(spleen tyrosine kinase) is a non-receptor tyrosine kinase, and present in the cell matrix. SYK is widely expressed in hematopoietic cells, lymphocytes, fibroblasts and vascular endothelial cells. It is highly expressed in B lymphocytes and plays an important role in tumors and autoimmune diseases. Dectin-1/ITAM is a classical pathway of antigen stimulated immune cells to induce immune diseases. In normal B cells, when antigen induced BCR crosslinks to phosphorylate the intracellular immune receptor tyrosinase activation motif (ITAM), SYK in the cytosol is the first target recruited and activated by ITAM, and the activated SYK then activates the transcription factor NF-κB through a CARD9 dependent pathway to produce a series of inflammatory factors. In addition, this pathway can also activate Caspase-8, and the activated Caspase-8 cleaves IL-1β precursor, promotes the immature IL-1β to maturation. CARD9-independent NLRP3 signaling pathway also plays a role in the maturation of the immature IL-1β. Therefore, SYK is also an important target of autoimmune diseases.

Psoriasis is an immune-mediated chronic, recurrent, inflammatory skin disease. The prevalence rate varies significantly across the world, ranging from 0.5% to 3.15% in the United States and 0.75% to 2.9% in Europe. China reported a psoriasis prevalence rate of 0.123% in 1984, a prevalence rate of 0.47% in six cities surveyed in 2008, and a prevalence rate of 0.5% in four provinces in Southwest China in 2017. There are more than 6 million psoriasis patients in China. Psoriasis can occur at any age without sex differences. About 2/3 of patients have onset before the age of 40, and most patients have severe symptom in winter and mild symptom in summer.

The etiology and pathogenesis of psoriasis have not yet been fully clarified, and may involve genetic, immune, environmental and other factors. Through the immune response mediated mainly by T lymphocytes and participated together by a variety of immune cells, psoriasis causes excessive proliferation of keratinocytes and inflammation of joint synoviocytes and chondrocytes. The typical clinical manifestation of psoriasis is scaly erythema or plaque, which is localized or widely distributed. Psoriasis can be associated with other systemic abnormalities, such as visceral and joint damage. Moderate to severe patients have an increased risk of metabolic syndrome and atherosclerotic cardiovascular disease.

According to the clinical manifestations and pathological characteristics of psoriasis, it can be divided into the following types: 1. Psoriasis vulgaris: the most common type and most being acute onset. The typical manifestation is erythema with clear boundaries and various shapes and sizes, which are surrounded by inflammatory flush. There is slight infiltration and thickening, and the surface is covered by multiple layers of silver-white scales. It is easy to scratch off the scales, and when the scales are cleaned off, there is a translucent thin film that is light red and bright. If the thin film is broken, slight bleeding can be observed (Auspitz sign). Skin lesions usually occur on the head, the pars sacralis and the extensor aspect of limbs. Some patients are self-conscious of different degrees of pruritus. 2. Pustular psoriasis: divided into generalized pustular psoriasis and palmar and plantar pustula psoriasis. Generalized pustular psoriasis is characterized by clusters of superficial sterile pustules on erythema, some of which may coalesce into pustular lakes. It can occur anywhere on the body and can be seen in the flexed and wrinkled parts of the limbs more commonly, and the oral mucosa can be involved at the same time. Acute onset or sudden exacerbation is often accompanied by systemic symptoms such as chills, pyrexia, arthrodynia, general malaise, and increased white blood cell count. It appears as periodic attacks and psoriasis vulgaris lesions often appear in the remission period. The palmoplantar pustulosis lesions are limited to hands and feet, occur symmetrically, are generally in good condition, the condition is stubborn, and occur repeatedly. 3. Erythrodermic psoriasis: also known as psoriatic exfoliative dermatitis. It is a serious psoriasis. It is often caused by external use of highly irritating medicaments, long-term massive application of glucocorticoids, and rapid reduction or sudden withdrawal of medicaments. It is manifested by diffuse flushing, swelling, and desquamation of the whole body's skin, accompanied by pyrexia, chilliness, discomfort, and other systemic symptoms, swelling of superficial lymph nodes, and increased white blood cell count. 4. Psoriasis arthropathica: also known as psoriatic arthritis. Psoriasis patients have rheumatoid arthritis-like joint damage at the same time, which can involve the whole body joints, but the terminal finger (toe) intersegmental joint disease is the most characteristic. The affected joints are red-swollen and painful, and the skin around the joints is often red-swollen. Joint symptoms are often aggravated or alleviated at the same time as skin symptoms. Blood rheumatoid factor is negative.

Nowadays, there is no specific therapy for psoriasis, and the main treatment methods include a local treatment, a physical treatment, a systemic treatment, a Chinese medicine treatment and other therapies. Among them, the local treatment includes a treatment with a medicament for external use, a physical therapy, and the like. The medicament for external use mainly includes vitamin D3 analogues, glucocorticoids, anthralin, tretinoin gel and cream, tars, immunosuppressants, and the like, and other medicaments for external use such as Tacrolimus, pimecrolimus, 0.03% camptothecin ointment, 5% salicylic acid ointment, and the like. The medicament for systemic treatment includes methotrexate, tretinoins, ciclosporin, tacrolimus, mycophenolate mofetil, biological agent such as etanercept, infliximab, and antibiotics. For moderate and severe patients, a combined, alternate or sequential treatment is usually given when a single treatment is not good. The current treatments have significant short-term effects but will produce many side effects together with high recurrence rate, and the long-term effects are not satisfatory, for example: the therapeutic dose of methotrexate is very close to its toxic dose; the main side effect of tretinoins is teratogenicity; the adverse effects of ciclosporin A include nephrotoxicity, hypertension, nausea, emesia, hypodynamia, amyostasia, urinary irritation symptoms and the like; the adverse effects of tacrolimus are similar to those of ciclosporin A; the adverse effects of mycophenolate mofetil include gastrointestinal symptoms, anemia, leucopenia, increased risk of infection and tumor induction, and the like.

Atopic dermatitis (AD), also referred to as atopic eczema and genetic allergic dermatitis, is an allergic skin disease characterized by skin pruritus and pleomorphic rash. It has different clinical manifestations at different ages. It is a chronic, recurrent, inflammatory skin disease. It is considered as a systemic disease since the patients are often concurrent with other atopic diseases such as allergic rhinitis and asthma. The prevalence of AD has gradually increased worldwide in the past 30 years. The prevalence of AD in children in developed countries has reached 10%-20%. The increase in the prevalence of AD in China is later than that in western developed countries, Japan and South Korea, but it has increased rapidly in the past 10 years. The overall prevalence of AD in school-age adolescents (6-20 years old) was 0.70% in 1998, the prevalence in preschool children (1-7 years old) in 10 cities was 2.78% in 2002, and the prevalence in children aged 3-6 years in Shanghai region was up to 8.3% in 2012. In 2014, the prevalence of AD in children aged 1-7 years in 12 cities in China reached 12.94%, and the prevalence of AD in infants aged 1-12 months reached 30.48%.

The pathogenesis of AD is closely related to genetic and environmental factors and the like. Although the exact pathogenesis is still unclear, it is currently believed that immunologic abnormality, skin barrier dysfunction, skin dysbacteriosis and other factors are important links in the pathogenesis. AD usually first occurs in infancy, patients attacked by AD before the age of 1 account for about 50% of all patients. It has a chronic course and a variety of clinical manifestations. The most basic features are xerosis cutis, chronic eczema-like skin lesions and obvious pruritus. Some patients may have other anaphylactic diseases at the same time, such as allergic asthma, allergic rhinoconjunctivitis, etc. In addition, due to the long-term chronic inflammatory reaction, patients with chronic disease course have significantly higher risk of concurrent nervous system disease, inflammatory bowel disease, rheumatoid arthritis, cardiovascular disease and lymphoma.

At present, the treatment of AD includes the basic treatment (avoiding contact allergy, etc.), the treatment with medicaments for external use (glucocorticoids for external use and calcineurin inhibitors for external use, etc.), systemic treatment (oral antihistamine medicaments, immunosuppressants, glucocorticoids, etc.), ultraviolet treatment and antimicrobial treatment, in which medicaments for external use, hormones, and immunosuppressants are predominant. A stepwise treatment mode is usually used to treat mild to moderate patients. Although the existing treatment methods can alleviate the symptoms, there are still some limitations and many adverse effects. There are still many unmet needs for prompt onset of action, pruritus control, and recurrence prevention. Lupus erythematosus (LE) is a typical autoimmune connective tissue disease, which can be divided into discoid lupus erythematosus (DLE), subacute cutaneous lupus erythematosus (SCLE), systemic lupus erythematosus (SLE), lupus erythematosus profundus (LEP), neonatal lupus erythematosus (NLE), medicament-induced lupus erythematosus (DIL) and other subtypes.

Discoid lupus erythematosus, which mainly invades the skin, is the lightest type of lupus erythematosus. A few cases may have mild visceral damage, a few cases may turn into systemic lupus erythematosus, and most patients have skin lesions without subjective symptoms, but it is difficult to completely subside. Subacute cutaneous lupus erythematosus is a special intermediate type, which is relatively rare clinically. Skin lesions often occur repeatedly. The vast majority of patients have visceral damage, but few are serious. The main symptoms are arthralgia, myalgia, and recurrent low fever. A minority of patients have nephritis and blood system changes. Lupus erythematosus profundus, also known as lupus panniculitis and lupus erythematosus profundus, is also an intermediate type of lupus erythematosus. It is unstable in nature and can exist alone. Later, it can be transformed into either discoid lupus erythematosus or systemic lupus erythematosus, or coexists with them. Neonatal lupus erythematosus, characterized by erythema annulare on the skin and congenital heart block, is self limiting and generally subsides within 4-6 months after birth. Cardiac lesions often persist. The main manifestations of medicament-induced lupus erythematosus are pyrexia, arthralgia, myalgia, facial butterfly erythema, dental ulcer, and possibly serositis, which gradually improves after medicament withdrawal. Patients with preponderant disease situations can be provided with medicament treatment. Systemic lupus erythematosus (SLE) is a systemic autoimmune disease, which is the most serious type of lupus erythematosus. It is characterized by systemic multi-system and multi-organ involvement, repeated recurrence and remission, and the presence of a large number of autoantibodies in the body. If not treated in time, it will cause irreversible damage to the affected organs and eventually lead to the death of patients. There are large regional differences in the prevalence of SLE. At present, the global prevalence of SLE is 0-241/ 100,000, the prevalence of SLE in the mainland of China is 30-70/100,000, and the prevalence ratio of male to female is 1:10-12. The specific pathogenic factors of SLE have not yet been fully defined. It is caused by the involvement of multiple factors, and mainly related to genetic, infection, endocrine, and environmental factors. Defects in immunoregulation mechanisms, such as the clearance of apoptotic cells and immune complexes, are also important factors in the occurrence of SLE. The lost immune tolerance, the increased antigen load, the excessive T cell assistance, the defective B cell suppression, and the transition from Th1 cells to Th2 cells lead to the hyperactivation of B cells and the production of pathogenic autoantibodies. In addition, some medicaments such as methyldopa, phenytoin sodium, penicillamine, quinidine, and propranolol can directly cause medicament-induced lupus and aggravate lupus erythematosus. The vast majority of SLE patients have multisystem damage manifestations at the onset. A minority of patients develop from other types of lupus erythematosus. Some patients also have other connective tissue diseases, such as scleroderma, dermatomyositis, and Sjogren's syndrome, forming various overlap syndromes.

At present, the medicament treatment of SLE is the medication based on glucocorticoids and hydroxychloroquines. However, the treatment with the above medicaments will produce corresponding side effects, such as infection, liver and kidney function damage and metabolic abnormalities, and sometimes have to reduce the amount used or stop the medicament. At present, there is still no good medicament to control the development of the disease, and it is still necessary to continue to develop new medicaments. SYK and JAK are respectively upstream of two different signaling pathways that induce SLE, so SYK-JAK dual channel inhibitors are expected to be an effective way to treat SLE. Currently, there is no JAK-SYK dual target inhibitor approved for marketing. R333, a JAK-SYK dual target inhibitor product with DLE as the development indication, has also been terminated due to a phase II clinical failure on October 24, 2013.

Therefore, there is a need to develop new medicaments for the treatment of autoimmune diseases, especially immune-mediated skin diseases and autoimmune connective tissue diseases such as psoriasis, atopic dermatitis or lupus erythematosus.

### Summary of the Invention

Compound (I) with the following structural formula (I): 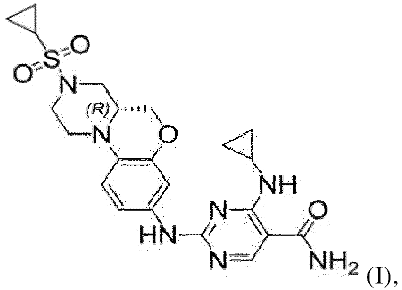 its chemical name is: (R)-4-(cyclopropylamino)-2- ((3-(cyclopropylsulfonyl)-1,2,3,4,4a,5-hexahydrobenzo[b]pyrazolo[1,2-d][1,4]oxazin-8-yl)amino)pyrimidine-5-carboxamide, which was first disclosed in the PCT international application with publication number of WO2018108084, and is known to be a highly selective JAK kinase and Syk kinase dual target inhibitor and can be used to treat cancer.

The present inventors have found through further research that Compound (I) plays a regulatory role in the cell signal transduction, division and proliferation by inhibiting JAK and SYK kinases, and has a superior effect in the treatment of autoimmune diseases, especially shows satisfactory activity and low toxicity and side effect in the treatment of immune-mediated skin diseases and autoimmune connective tissue diseases such as psoriasis, atopic dermatitis and lupus erythematosus.

The present invention provides the use of Compound (I), an optical isomer thereof or a pharmaceutically acceptable salt thereof in manufacture of a medicament for treating a disease related to high expression or abnormal activation of JAK and SYK kinases:

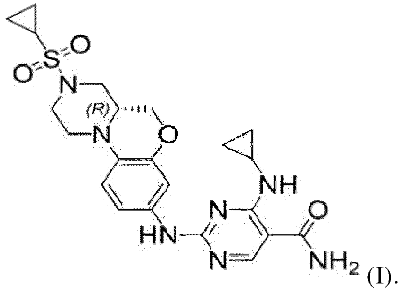

Preferably, in the above-mentioned use, the JAK kinase is one or more of JAK1, JAK2, JAK3 or TYK2 kinase, preferably JAK3 and/or TYK2 kinase.

Preferably, in the above-mentioned use, the disease related to high expression or abnormal activation of JAK and SYK kinase is an autoimmune disease.

Preferably, in the above-mentioned use, the autoimmune disease is an immune-mediated skin disease or an autoimmune connective tissue disease.

Preferably, in the above-mentioned use, the immune-mediated skin disease is psoriasis or atopic dermatitis, the psoriasis is preferably psoriasis vulgaris, pustular psoriasis, erythroderma psoriaticum or psoriasis arthropathica; the autoimmune connective tissue disease is lupus erythematosus, the lupus erythematosus is preferably discoid lupus erythematosus, subacute cutaneous lupus erythematosus, systemic lupus erythematosus, lupus erythematosus profundus, neonatal lupus erythematosus, medicament-induced lupus erythematosus, further preferably systemic lupus erythematosus.

Further, in the above-mentioned use, the medicament contains a therapeutically effective amount of Compound (I), an optical isomer thereof or a pharmaceutically acceptable salt thereof, and optionally a pharmaceutically acceptable excipient or carrier.

There are no special restrictions on the administration mode of the medicament of the present invention, and the representative administration mode includes but is not limited to oral, rectal, parenteral (intravenous, intramuscular or subcutaneous) and topical administration. Accordingly, the medicament of the present invention may be made into various clinically acceptable dosage forms, including oral dosage forms, injection dosage forms, topical dosage forms, external dosage forms, or the like.

Preferably, the medicament of the present invention may be clinically used alone or in combination with other therapeutic component(s). To facilitate the clinical use, Compound (I) , an optical isomer thereof or a pharmaceutically acceptable salt thereof of the the present invention can be combined with other therapeutic component(s) to prepare compound medicaments or combination products.

The present invention also provides a method for using the medicament, that is, applying a therapeutically effective amount of Compound (I) , an optical isomer thereof or a pharmaceutically acceptable salt thereof of the present invention to a mammal (such as human) in need thereof.

The present invention provides a method for treating a disease related to high expression or abnormal activation of JAK and SYK kinases, which is characterized by applying a therapeutically effective amount of Compound (I) , an optical isomer thereof or a pharmaceutically acceptable salt thereof of the present invention to a mammal (such as human) in need thereof.

The JAK kinase of the present invention is one or more of JAK1, JAK2, JAK3 or TYK2 kinase, preferably JAK3 and/or TYK2 kinase. The disease related to high expression or abnormal activation of JAK and SYK kinase of the present invention is an autoimmune disease, such as an immune-mediated skin disease and an autoimmune connective tissue disease, the immune-mediated skin disease is psoriasis or atopic dermatitis, the psoriasis is preferably psoriasis vulgaris, pustular psoriasis, erythroderma psoriaticum or psoriasis arthropathica; the autoimmune connective tissue disease is lupus erythematosus, the lupus erythematosus is preferably discoid lupus erythematosus, subacute cutaneous lupus erythematosus, systemic lupus erythematosus, lupus erythematosus profundus, neonatal lupus erythematosus, medicament-induced lupus erythematosus, further preferably systemic lupus erythematosus.

The present invention also provides a medicament containing a therapeutically effective amount of compound (I), an optical isomer thereof or a pharmaceutically acceptable salt thereof, which is characterized in that the medicament is useful for treating a disease related to high expression or abnormal activation of JAK and SYK kinases in a subject, wherein said disease is an autoimmune disease, such as an immune-mediated skin disease and an autoimmune connective tissue disease, the immune-mediated skin disease is psoriasis or atopic dermatitis, the psoriasis is preferably psoriasis vulgaris, pustular psoriasis, erythroderma psoriaticum or psoriasis arthropathica; the autoimmune connective tissue disease is lupus erythematosus, the lupus erythematosus is preferably discoid lupus erythematosus, subacute cutaneous lupus erythematosus, systemic lupus erythematosus, lupus erythematosus profundus, neonatal lupus erythematosus, medicament-induced lupus erythematosus, further preferably systemic lupus erythematosus.

The JAK kinase is one or more of JAK1, JAK2, JAK3 or TYK2 kinase, preferably JAK3 and/or TYK2 kinase.

The present invention also provides a compound medicament or combination product, containing a therapeutically effective amount of Compound (I), an optical isomer thereof or a pharmaceutically acceptable salt thereof, and other therapeutic component(s), which is characterized in that the compound medicament or combination product is useful for treating a disease related to high expression or abnormal activation of JAK and SYK kinases in a subject, wherein said disease is an autoimmune disease, such as an immune-mediated skin disease and an autoimmune connective tissue disease, the immune-mediated skin disease is psoriasis or atopic dermatitis, the psoriasis is preferably psoriasis vulgaris, pustular psoriasis, erythroderma psoriaticum or psoriasis arthropathica; the autoimmune connective tissue disease is lupus erythematosus, the lupus erythematosus is preferably discoid lupus erythematosus, subacute cutaneous lupus erythematosus, systemic lupus erythematosus, lupus erythematosus profundus, neonatal lupus erythematosus, medicament-induced lupus erythematosus, further preferably systemic lupus erythematosus.

The JAK kinase is one or more of JAK1, JAK2, JAK3 or TYK2 kinase, preferably JAK3 and/or TYK2 kinase.

The therapeutically effective amount mentioned in the present invention refers to the pharmaceutically effective administration dose, that is, the amount of the active compound is sufficient to significantly improve the condition without causing serious side effects. For a person with a body weight of 60 kg, the daily administration dose is usually 0.01-2000 mg, preferably 1-500 mg, more preferably 10-400 mg, further preferably 15-360 mg or 15-250 mg, such as 15 mg, 45 mg, 60 mg, 90 mg, 135 mg, 180 mg, 240 mg, 300 mg, 360 mg. It can be administered in a single dose once a day, in multiple doses in a day, or at intervals. For the specific dose and the frequency of administration, the factors such as the route of administration and the health status of patients should be considered, and these can be determined by skilled physicians according to routine skills. There are no special restrictions on the administration mode, and the representative administration mode includes but is not limited to oral, rectal, parenteral (intravenous, intramuscular or subcutaneous) and topical administrations. The amount of the active compound is calculated as Compound (I).

In vivo and in vitro studies show that (1) Compound (I) of the present invention can significantly inhibit the kinase activities of JAK1, JAK2, JAK3, TYK2, and SYK in vitro, and has strong inhibitory effects on JAK3 and TYK2, with IC₅₀s of 1.43 nM and 0.82 nM respectively. The inhibitory effects on JAK2 and SYK are slightly weaker than those on JAK3 and TYK2, with IC₅₀s of 3-8 nM. The inhibitory effect on JAK1 is the weakest, with an IC₅₀ of 20.04 nM. (2) Compound (I) of the present invention can improve or significantly reduce the skin thickness, the ear thickness, the spleen weight, the spleen index, the PASI scores (red swelling, scale, thickness and total score), the mouse skin epidermal thickness, the skin pathology score, and the content of IL-6 and TNF-α in the skin tissue of the IMQ-induced psoriasis model mice. (3) Compound (I) of the present invention can improve or significantly reduce the skin thickness and the skin clinical score of oxa induced atopic dermatitis model mice; and can improve the skin inflammatory cell aggregation, the edema and the telangiectasia in the modeling area, and significantly reduce the pathological score and the epidermal thickness. (4) Compound (I) of the present invention can dose-dependently improve the skin lesions of MRL/lpr lupus erythematosus model mice, alleviate the kidney injury, reduce the enlargement of the lymph nodes and spleen, and inhibit the increase of the SLE related antibodies and cytokines in the serum. Among others, after 7 weeks of the administration in the 20 mg/kg group of mice, the lymphadenectasis of the SLE mice can be effectively inhibited. The indicator evaluation results at the end of the test show that the administration at 20 mg/kg can effectively inhibit the splenomegaly and the lymphadenectasis, and the total pathological score of double kidneys in the chronic index (CI) decreases significantly, and the overexpression of IL-6 and TNF-alpha in the serum can also be inhibited. The administration at doses of 40 mg/kg and 60 mg/kg can comprehensively improve various symptoms of the SLE mice, including improving the skin lesions (the skin destruction score and the skin pathological score), alleviating the kidney injury (reducing the mouse urine protein and decreasing the kidney immune complex deposition), and inhibiting the immune organ enlargement (improving the lymph node pathological score and inhibiting the spleen and lymph node enlargement).

The above research results show that Compound (I) , an optical isomer thereof or a pharmaceutically acceptable salt thereof of the present invention can improve the skin lesions of the psoriasis mice and the atopic dermatitis mice and inhibit the immune organ enlargement; can also dose-dependently improve the skin lesions of the SLE mice, inhibit the immune organ enlargement, alleviate the renal function damage, reduce the level of inflammation, and has a certain safe therapeutic window, resulting in a good clinical application prospect.

### Brief description of the drawings

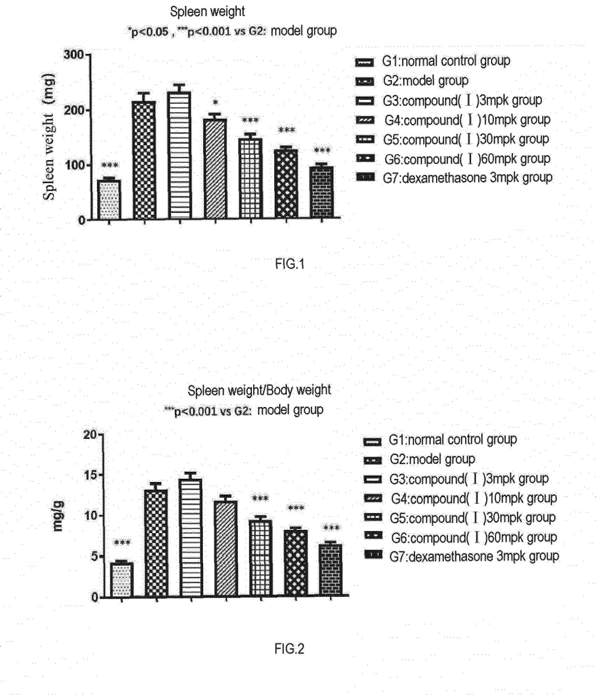

Figure 1: a diagram for the spleen weight of the IMQ-induced psoriasis model mice at the end of the study.
Figure 2: a diagram for the spleen index (spleen weight/body weight%) of the IMQ-induced psoriasis model mice at the end of the study.

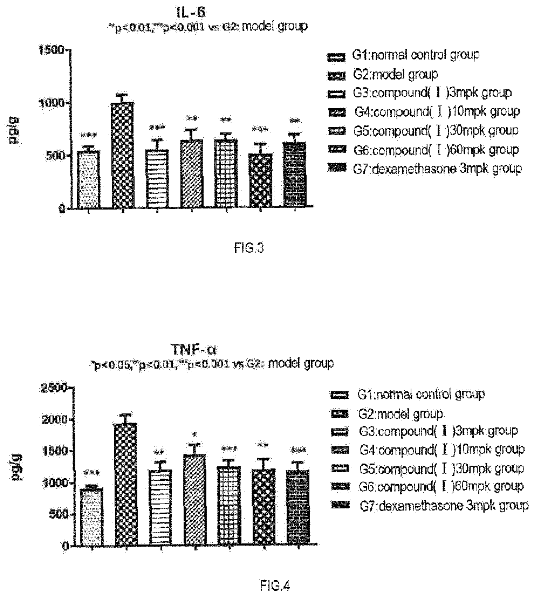

Figure 3: a diagram for the content of IL-6 in the mouse skin tissue of the IMQ-induced psoriasis model mice at the end of the study.
Figure 4: a diagram for the content of TNF-alpha in the mouse skin tissue of the IMQ-induced psoriasis model mice at the end of the study.

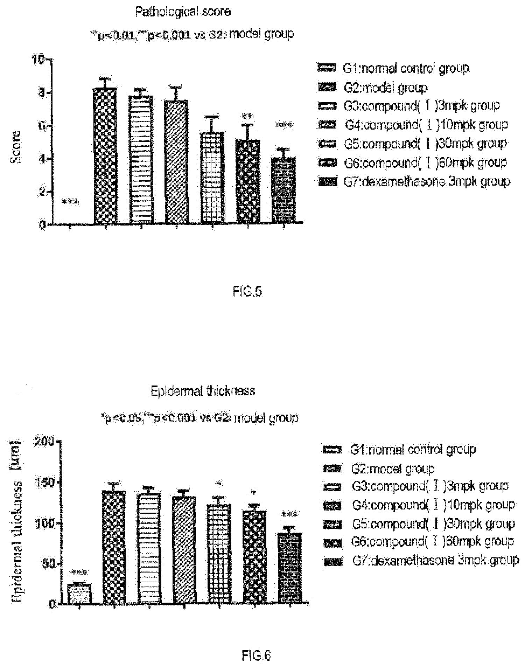

Figure 5: a diagram for the skin pathological score of the IMQ-induced psoriasis model mice at the end of the study.
Figure 6: a diagram for the skin epidermal thickness of the IMQ-induced psoriasis model mice at the end of the study.

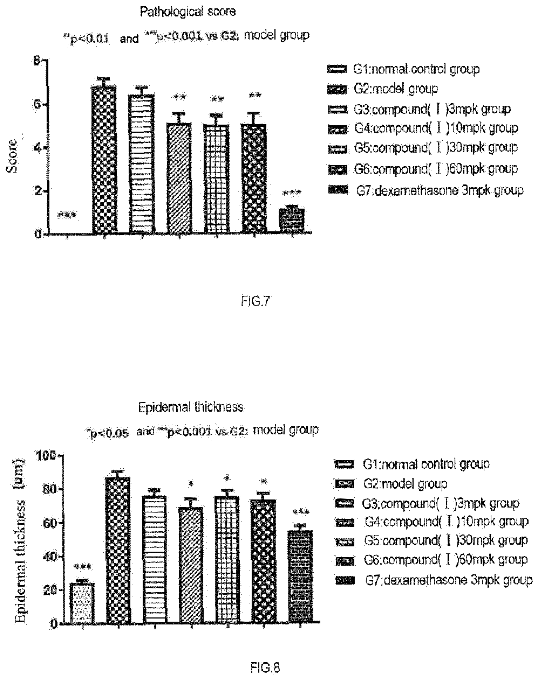

Figure 7: a diagram for the skin pathological score of the OXA-induced atopic dermatitis model mouse at the end of the study.
Figure 8: a diagram for the skin epidermal thickness of the OXA-induced atopic dermatitis model mouse at the end of the study.
Figure 9: a diagram for the skin pathological score of the SLE mice at the end of the test.
Figure 10: a diagram for the lymph node weight of the SLE mice at the end of the test, A: total lymph node weight; B: total lymph node weight/body weight%.
Figure 11: a diagram for the spleen weight of the SLE mice at the end of the test, A: spleen weight; B: spleen weight/body weight%.
Figure 12: a diagram for the area under the urinary protein curve of the SLE mice that have been treated for 16 weeks.
Figure 13: a diagram for the kidney weight of the SLE mice at the end of the test, A: total kidney weight; B: kidney weight/body weight%.
Figure 14: a diagram for the kidney tissue HE staining score of the SLE mice, A: bilateral kidney HE score - activity index; B: bilateral kidney HE score - chronicity index; C: bilateral kidney HE score - renal tubule interstitial injury.
Figure 15: a diagram for the kidney tissue IHC (IgG) staining score of the SLE mice.
Figure 16: a diagram for the serum anti-ds-DNA antibody concentration of the SLE mice.
Figure 17: a diagram for the serum cytokine level of the SLE mice, A: TNF-alpha concentration; B: IL-6 concentration.

### Detailed description

The present invention is further described below in combination with specific examples. It should be understood that these examples are only used to illustrate the present invention and not to limit the scope of the invention. The experimental methods for which no specific conditions are indicated in the following examples are generally based on conventional conditions or according to conditions recommended by the manufacturer.

Source or preparation of experimental materials:
1. Compound (I): self-prepared by CSPC Zhongqi Pharmaceutical Technology (Shijiazhuang) Co. Ltd..
2. Positive control medicaments, reagents used in the experiment, and raw materials were commercially available or self-prepared.
3. Preparation method of test substances for in vivo experiments (Compound (I) and positive control compounds):
   Each substance was weighed and then dissolved in an aqueous solution containing 0.4% Tween 80 and 0.5% methylcellulose; Compound (I) was prepared into the concentrations of 0.3 mg/mL, 1 mg/mL, 2 mg/mL, 3 mg/mL, 4 mg/mL and 6 mg/mL respectively; and the positive control medicaments were prepared with normal saline into the concentration of 0.3 mg/mL or 0.6 mg/mL, and diluted to the required concentration just before the use.
4. Vehicle (0.4% Tween 80/0.5% methylcellulose) prepared into 1 L:
   5.0 g of methylcellulose powder was weighed and added into a clean glass bottle, 900 mL of sterile water was added, and the mixture was stirred overnight until fully dissolved; 4.0 mL of Tween 80 was suctioned, and the mixture was fully stirred and uniformly mixed. The volume was fixed to the final volume of 1000 mL. The solution was preserved in a refrigerator at 4°C.
5. Preparation of OXA:
   Preparation of acetone/olive oil (4/1) vehicle: 40 mL of acetone and 10 mL of olive oil were mixed and oscillated for 30 seconds until mixed uniformly to produce the acetone/olive oil (4/1) vehicle.
   Preparation of 5% OXA: 100.00 mg of OXA was weighed, dissolved in 2.00 mL of acetone/olive oil (4/1), and oscillated for 30 seconds.
   Preparation of 0.1% OXA: 15.69 mg of OXA was weighed, dissolved in 15.69 mL of acetone/olive oil (4/1), and oscillated for 30 seconds. It was prepared when used and prepared once every three days.

### Example 1: kinase activity inhibition test of Compound (I)

1. kinases: SYK, JAK1, JAK2, JAK3 and TYK2
2. test method
Protein kinase activity was determined by Mobility Shift Assay. Compound (I) was dissolved in DMSO and prepared into concentration gradients in 100% DMSO. By the dilution with the kinase buffer, 5 µL of Compound (I) (10% DMSO) was added to a 384-well plate at 5 folds of the final reaction concentration. 10 µL of 2.5-fold enzyme solution was added and the incubation was performed at room temperature for 10 min, followed by the addition of 10 µL of 2.5-fold substrate solution. The incubation was performed at 28°C for 60 min and 30 µL of stop solution was added to the 384-well reaction plate to terminate the reaction (100 mM HEPES, pH 7.5, 0.015% Brij-35, 0.2% Coating Reagent #3, 50 mM EDTA). The conversion data was copied from the Caliper EZ Reader, and converted into the inhibition data, %inhibition=(max-conversion)/(max-min)×100%, wherein, "min" is the conversion for the control sample well where the reaction was performed without adding the enzyme; and "max" is the conversion for the control well where DMSO was added. The curve was drawn with the compound concentration and the inhibition rate as the horizontal and vertical coordinates, and the IC₅₀ value was calculated by fitting with XLFit excel add-in version 5.4.0.8. Fitting equation: Y=Bottom + (Top-Bottom)/(1+(IC₅₀/X) ^HillSlope).

| Kinase | Kinase Buffer | Substrate |
|---|---|---|
| SYK | 20 mM HEPES, pH 7.5, 0.01% Triton X-100 | FAM-Peptide 22 and 2 µM ATP |
| JAK1 | 25 mM HEPES, pH 7.5, 0.01% Brij-35, 0.01% Triton, 0.5 mM EGTA | FAM-Peptide D and 66 µM ATP |
| JAK2 | | FAM-Peptide 22 and 6.5 µM ATP |
| JAK3 | | FAM-Peptide 22 and 4.3 µM ATP |
| TYK2 | | FAM-Peptide 30 and 16 µM ATP |

3. test result

The test result for the kinase inhibitory activity of Compound (I) was shown in the table below:

**Table 1: Kinase inhibitory activity of Compound (I)**

| Kinase | JAK1 | JAK2 | JAK3 | TYK2 | SYK |
|---|---|---|---|---|---|
| IC₅₀, nM | 20.04 | 3.92 | 1.43 | 0.82 | 7.25 |

### Example 2: pharmacodynamic test of Compound (I) in the IMQ-induced mouse psoriasis model

### 1. Test purpose

The purpose of this test was to evaluate the pharmacodynamics of Compound (I) in the IMQ (5% imiquimod cream cream)-induced mouse psoriasis model. One of the clinical adverse effects of imiquimod is to induce the onset of psoriasis. The imiquimod-induced mouse psoriasis model is simple to operate and easy to implement, and possesses the following advantages: complex interactions between tissues exist for skin phenotype and pathological characteristics; the model is similar to clinical psoriasis.

### 2. Test medicament

Test medicament: Compound (I)
Positive control medicament: dexamethasone (Dex)
Vehicle: 0.4% Tween 80/0.5% methylcellulose.

### 3. Test animal

female Balb/c mice aged 5-6 weeks, number: 70.

### 4. Test grouping and dosage regimen

The animals were randomly divided into seven groups according to the body weights on the day before the start of the experiment. See Table 2 for details:

**Table 2: Grouping of psoriasis pharmacodynamic test**

| Group | Amount | Modeling | Administration | Route and Frequency of Administration | Dose of Administration (mpk) | Duration of Administration | Study Endpoint |
|---|---|---|---|---|---|---|---|
| 1 | 10 | Normal Control | Vehicle | PO bid | / | 14 days | 14^{th} Day |
| 2 | 10 | IMQ Induction | Vehicle | PO bid | / | 14 days | 14^{th} Day |
| 3 | 10 | IMQ Induction | Compound (I) | PO bid | 3 | 14 days | 14^{th} Day |
| 4 | 10 | IMQ Induction | Compound (I) | PO bid | 10 | 14 days | 14^{th} Day |
| 5 | 10 | IMQ Induction | Compound (I) | PO bid | 30 | 14 days | 14^{th} Day |
| 6 | 10 | IMQ Induction | Compound (I) | PO bid | 60 | 14 days | 14^{th} Day |
| 7 | 10 | IMQ Induction | Dex | PO qd | 3 | 14 days | 14^{th} Day |

| | | | | | | | |
|---|---|---|---|---|---|---|---|
| Note: "po" means oral; "bid" means twice a day; "qd" means once a day; mpk: mg/kg. | | | | | | | |

### Modeling:

The animals were shaved on their back one day before applying the medicament, forming a skin exposure area having a size of about 2 cm × 3 cm. The mice were induced by IMQ, and applied with 62.5 mg of 5% imiquimod cream on the bare back and the right ear at regular intervals every day for 14 days. Mice in the normal control group were applied with vaseline on the bare back and the right ear at regular intervals every day.

### 5. Test result

### 5.1 Influence of Compound (I) on the skin thickness of the IMQ-induced mice

The skin thickness was measured daily from the first day of the test until the end of the test. The skin thickness measurement method was as follows: the back skin and the subcutaneous tissue of the mouse were pinched with the thumb and the index finger of the left hand in the same direction as the mouse body; the skinfold thickness was measured 1 cm away from the pinched part of the left hand (near the center of the molding part) with a digital thickness gauge (with millesimal reading) held in the right hand (model BK-3281, manufacturer: Shanghai Niuhui). The thickness was in mm, and the actual thickness was the half of the measured thickness (the skin thickness = the measured value /2).

The test results showed that compared with the normal control group, the skin thickness of mice in the model group was significantly increased after being stimulated with 5% imiquimod cream for 14 consecutive days. At the end point of the study, the treatment with Compound (I) (3 mpk, 10 mpk, 30 mpk and 60 mpk) and dexamethasone (3 mpk) significantly inhibited the increase of the skin thickness (all 5 groups: p<0.001 vs the model group). See Table 3 for details.

**Table 3: Skin thickness of IMQ-induced mice after 14 days of treatment (mean±standard error, unit: mm)**

| Group | 1^{st} Day | 14^{th} Day |
|---|---|---|
| 1 | 0.34 ± 0.00 | 0.26 ± 0.00*** |
| 2 | 0.33 ± 0.00 | 0.60 ± 0.02 |
| 3 | 0.32 ± 0.00 | 0.51 ± 0.01*** |
| 4 | 0.32 ± 0.00 | 0.53 ± 0.02*** |
| 5 | 0.32 ± 0.00 | 0.50 ± 0.01*** |
| 6 | 0.33 ± 0.01 | 0.43 ± 0.02*** |
| 7 | 0.32 ± 0.00 | 0.32 ± 0.01*** |

| | | |
|---|---|---|
| Note: **P*<0.05, ***P*<0.01, ****P*<0.001 vs the model group. | | |

### 5.2 Influence of Compound (I) on the ear thickness of the IMQ-induced mice

The ear thickness was measured daily (at the center of the auricle).

The test results showed that compared with the normal control group, the stimulation with 5% imiquimod cream for 14 consecutive days significantly increased the right ear thickness of mice. At the end point of the study, the treatment with Compound (I) (3 mpk, 10 mpk, 30 mpk and 60 mpk) and dexamethasone (3 mpk) significantly inhibited the increase of the ear thickness (all 5 groups: p<0.001 vs the model group). Compound (I) inhibited the increase of the ear thickness in a dose-dependent manner. See Table 4 for details.

**Table 4: Ear thickness of IMQ-induced mice after 14 days of treatment (mean±standard error, unit: mm)**

| Group | 1^{st} Day | | | 14^{th} Day | | |
|---|---|---|---|---|---|---|
| | Right | Left | Right-Left | Right | Left | Right-Left |
| 1 | 0.15 ± 0.00 | 0.15 ± 0.00 | 0.00 ± 0.00 | 0.15 + 0.00*** | 0.15 ± 0.00 | 0.00±0.00*** |
| 2 | 0.15 ± 0.00 | 0.15 ± 0.00 | 0.00 ± 0.00 | 0.54 + 0.00 | 0.16 ± 0.00 | 0.38 ± 0.00 |
| 3 | 0.15 ± 0.00 | 0.15 ± 0.00 | 0.00 ± 0.00 | 0.48 + 0.01*** | 0.16 ± 0.00 | 0.32±0.01*** |
| 4 | 0.14 ± 0.00 | 0.14 ± 0.00 | 0.00 ± 0.00 | 0.44 + 0.00*** | 0.15 ± 0.00 | 0.28 ± 0.00 |
| 5 | 0.14 ± 0.00 | 0.14 ± 0.00 | 0.00 ± 0.00 | 0.41 ± 0.01*** | 0.16 ± 0.00 | 0.26 ± 0.01*** |
| 6 | 0.14 ± 0.00 | 0.14 ± 0.00 | 0.00 ± 0.00 | 0.38 ± 0.01*** | 0.15 ± 0.00 | 0.22 ± 0.01*** |
| 7 | 0.14 ± 0.00 | 0.14 ± 0.00 | 0.00 ± 0.00 | 0.33 ± 0.01*** | 0.15 ± 0.00 | 0.18 ± 0.00*** |

| | | | | | | |
|---|---|---|---|---|---|---|
| Note: **P*<0.05, ***P*<0.01, ****P*<0.001 vs G2 (model group). | | | | | | |

### 5.3: Influence of Compound (I) on the skin PASI score of IMQ-induced mice

Skin photographs were taken daily from the first day of the test until the end of the test. The clinical symptoms were evaluated with the PASI evaluation standard from three indicators of the erythema (Erythema), the scale (Scaling) and the skin thickness (Thickness), and scored with 0-4 points. The total score was obtained by summing the three scores. The PASI evaluation standard was as follows: 0, asymptomatic; 1, mild; 2, moderate; 3, severe; 4, extremely severe.

Erythema: 0- no erythema visible; 1- light red; 2- red; 3- dark red; 4- extremely dark red.

Scaling: 0- no visible scale on the skin surface; 1- part of the skin lesion surface covered with scales; 2- most of the skin lesion surface covered with scales; 3- almost all of the skin lesion region covered with scales; 4- all of the skin lesion region covered with scales. Skin thickness: 0- smooth skin without wrinkles; 1- slight wrinkles at the edges of the skin, or the rough skin; 2- slight wrinkles or slight humps appeared at all of skin lesions; 3- the wrinkle degree of the skin lesion was further deepened, or the skin lesion was significantly thickened with obvious humps; 4- the skin lesion was completely wrinkled, or the skin lesion was significantly thickened with obvious protuberances.

Dryness and pruritus are not included in the total score.

The test results showed that the PASI scores of the erythema, the scale, the skin thickness and the total score of the mice in the model group were significantly increased after being stimulated with 5% imiquimod cream for 14 consecutive days. At the end point of the study, the treatment with Compound (I) (3 mpk) significantly improved the scale score (p<0.001 vs the model group) and the total score (p<0.05 vs the model group); the treatment with Compound (I) (10 mpk and 30 mpk) significantly improved the scale score (the group of 10 mpk: p<0.01 vs the model group; the group of 30 mpk: p<0.001 vs the model group), the skin thickness score (both groups: p<0.001 vs the model group) and the total score (the group of 10 mpk: p<0.01 vs the model group; the group of 30 mpk: p<0.001 vs the model group); The treatment with Compound (I) (60 mpk) and dexamethasone (3 mpk) significantly improved the erythema score, the scale score, the skin thickness score and the total score (both groups: p<0.001 vs the model group). See Table 5 for details.

**Table 5: Skin PASI scores of IMQ-induced mice after 14 days of treatment (mean±standard error)**

| Group | 1^{st} Day | | | | 14^{th} Day | | | |
|---|---|---|---|---|---|---|---|---|
| | Erythema | Scaling | Thickness | Total Score | Erythema | Scaling | Thickness | Total Score |
| 1 | 0.00 ± 0.00 | 0.00 ± 0.00 | 0.00 ± 0.00 | 0.00 ± 0.00 | 0.00 ± 0.00*** | 0.00 ± 0.00" | 0.00±0.00*** | 0.00 ± 0.00" |
| 2 | 0.00 ± 0.00 | 0.00 ± 0.00 | 0.00 ± 0.00 | 0.00 ± 0.00 | 3.00 ± 0.00 | 3.00 ± 0.00 | 3.00 ± 0.00 | 9.00 ±0.00 |
| 3 | 0.00 ± 0.00 | 0.00 ± 0.00 | 0.00 ± 0.00 | 0.00 ± 0.00 | 3.00. ± 000 | 2.30 ±0.15*** | 2.80 ± 0.13 | 8.10 ±0.23* |
| 4 | 0.00 ± 0.00 | 0.00 ± 0.00 | 0.00 ± 0.00 | 0.00 ± 0.00 | 2.90 ± 0.10 | 2.40±0.27** | 2.40±0.27*** | 7.70 ±0.60** |
| 5 | 0.00 ± 0.00 | 0.00 ± 0.00 | 0.00 ± 0.00 | 0.00 ± 0.00 | 2.90 ± 0.10 | 1.2 ±0.13*** | 1.40±0.16*** | 5.50 ± 0.31*** |
| 6 | 0.00 ± 0.00 | 0.00 ± 0.00 | 0.00 ± 0.00 | 0.00 ± 0.00 | 2.00±0.15*** | 1.20±0.13*** | 1.40±0.16*** | 4.60±0.34*** |
| 7 | 0.00 ± 0.00 | 0.00 ± 0.00 | 0.00 ± 0.00 | 0.00 ± 0.00 | 2.10±0.10*** | 1.10±0.10*** | 0.90±0.10*** | 4.10±0.18*** |

### 5.4 Influence of Compound (I) on the spleen weight of the IMQ-induced mice

On the 14th day of the test, the spleen weight was collected.

The test results showed that the treatment of Compound (I) (10 mpk, 30 mpk and 60 mpk) and dexamethasone (3 mpk) significantly inhibited the increase in the spleen weight (the group of 10 mpk of Compound (I): p<0.05 vs the model group; all of the other three groups: p<0.001 vs the model group); and the treatment of Compound (I) (30 mpk and 60 mpk) and dexamethasone (3 mpk) significantly inhibited the increase in the spleen index (all of the three groups: p<0.001 vs the model group). See Table 6 for details.

**Table 6: Spleen weight and Spleen index (spleen weight/body weight%) of IMQ-induced mice after 14 days of treatment**

| (mean±standard error) | | |
|---|---|---|
| Group | Spleen weight (mg) | Spleen weight/body weight (mg/g) |
| 1 | 73.11 ± 3.20 | 4.24 ± 0.19 |
| 2 | 216.20 ± 13.22 | 13.12 ± 0.77 |
| 3 | 231.44 ± 12.72 | 14.46 ± 0.71 |
| 4 | 182.01 ± 9.11* | 11.71 ± 0.56 |
| 5 | 146.81 ± 7.71*** | 9.27 + 0.47*** |
| 6 | 125.73 ± 4.63*** | 8.01 ± 0.28*** |
| 7 | 93.79 ± 4.64*** | 6.17 ± 0.29*** |

| | | |
|---|---|---|
| Note: **P*<0.05, ***P*<0.01, ****P*<0.001 vs G2 (model group) | | |

### 5.5 Skin epidermal thickness measurement and pathological score

After the test, all mice were euthanized by excessive inhalation of carbon dioxide. The skin was collected and divided into four parts, one of which was fixed in a tissue fixative solution for 24 hours for the pathological detection. It was dehydrated, then embedded in paraffin and made into 4-micron sections. Skin sections were stained with hematoxylin eosin to observe the infiltration of the stratum corneum, the epidermal layer, the dermis layer and the inflammatory cell. When measuring the thickness of the epidermal layer, the stained skin sections were scanned with a Leca Aperio CS2 scanner at 200×magnification, and then the histopathological changes were observed and scored.

The specific criteria of the pathological score were as follows: munro microabscess found in the epidermal layer 2.0 points; hyperkeratosis 0.5 points; parakeratosis 1.0 points; the thinning or disappearing of the granular layer 1.0 points; the thickening of acanthosis 1.0 points; the extension and fluctuation of cuticular process 0.5, 1.0 and 1.5 points respectively according to the mild, moderate and severe degrees. The monocyte or coenocyte infiltration in the dermis layer 0.5, 1.0 and 1.5 points respectively according to the mild, moderate and severe degrees; the extension of mastoid process 0.5 points; and telangiectasia 0.5 points.

When measuring the thickness of the epidermal layer, the stained skin sections were scanned with a Leca Aperio CS2 scanner at 200×magnification, and then the scanned images were opened with HALO analysis software. A "typing" template in the software was used to define the skin epidermis as an annotation layer, the epidermis was divided into upper and lower segments in the annotation layer, and then about 200 thickness values in the segments were calculated with the "layer thickness" option. The epidermal thickness of each section was represented by the average value of epidermal thickness.

The test results indicated that Group 1, the normal control group showed that the intact skin structure was visible under the microscope and the cell morphology was normal without obvious abnormal changes. The skin of Group 2, the model group, showed visible Munro microabscess, hyperkeratosis or parakeratosis, and acanthosis thickening, accompanied by obvious hemangiectasis, and moderate to severe inflammatory cell infiltration. Compared with Group 1, the normal control group, the stimulation with 5% imiquimod cream for 14 consecutive days significantly increased the pathological score of skin tissue. The treatment with Compound (I) (60 mpk) and dexamethasone (3 mpk) significantly improved the pathological score of the skin tissue of the model mouse (the group of 60 mpk of Compound (I): p<0.01 vs the model group; the dexamethasone group: p<0.001 vs the model group). Compared with Group 1, the normal control group, the skin epidermal thickness of Group 2, the model group significantly increased. The treatment with Compound (I) (30 mpk and 60 mpk) and dexamethasone (3 mpk) significantly reduced the epidermal thickness of the model mouse (Group 2 of Compound (I): p<0.05 vs the model group; the dexamethasone group: p<0.001 vs the model group). See Table 7 for details.

**Table 7: Pathological score and epidermal thickness of IMQ-induced mice after 14 days of treatment (mean±standard error)**

| Group | Pathological score | Epidermal thickness (µm) |
|---|---|---|
| 1 | 0.00 ± 0.00 | 25.32 ± 0.93 |
| 2 | 8.25 ± 0.56 | 138.78 ± 9.69 |
| 3 | 7.75 ± 0.39 | 136.10 ± 5.96 |
| 4 | 7.45 ± 0.80 | 131.63 ± 7.15 |
| 5 | 5.55 ± 0.90 | 121.75 ± 8.17* |
| 6 | 5.05 ± 0.88** | 113.28 ± 6.95* |
| 7 | 3.95 ± 0.50*** | 84.98 ± 7.40*** |

| | | |
|---|---|---|
| Note: **P*<0.05, ***P*<0.01, ****P*<0.001 vs G2 (model group) | | |

### 5.6 Detection of inflammatory factors in skin samples

After the test, all mice were euthanized by excessive inhalation of carbon dioxide (CO2), and the skin was collected and divided into four parts, three of which were quickly frozen in liquid nitrogen and stored in a -80° C refrigerator for the inflammatory factor detection. IL-6 and TNF-alpha were detected by the ELISA method.

Tissue specimen: a certain amount of skin tissue was weighed, a certain amount of PBS (pH=7.4) was added, and the specimen was sufficiently homogenized by hand or with a homogenizer. The specimen was centrifuged for 20 min (2000-3000 rpm). The supernatant was carefully collected. After subpackaging, one was to be tested, and the rest was frozen for standby.

Operation steps:
1. Addition of standards: Standard wells and sample wells were set, and 50 µL of standards at different concentrations were added to standard wells.
2. Addition of samples: blank wells (neither a sample nor an ELISA reagent was added to a blank control well, but the rest steps were identical to those for the blank well) and sample-to-be-measured wells were set. To the sample-to-be-measured well of the ELISA coating plate were added firstly 40µL of the sample dilution and then 10µL of the sample to be measured (the final sample dilution degree: 5 folds). The sample was added to the bottom of the well of the ELISA plate, but not touched to the wall of the well as much as possible, and gently shaked and mixed uniformly.
3. Addtion of an enzyme: 100µL of an ELISA reagent was added to each well except for the blank well.
4. Incubation: The plate was sealed with a microplate-sealing membrane for incubation for 60 minutes at 37°C.
5. Solution preparation: A 20-fold concentrated washing solution was diluted by 20 folds with distilled water for standby.
6. Washing: The microplate-sealing membrane was carefully removed, and a liquid was discarded. The plate was spin-dried. Each well was filled with a washing solution and left by standing for 30 seconds, and then the washing solution was removed. This process was repeated for 5 times, and then the plate was patted to dryness.
7. Color development: To each well were first added 50µL of developer A and then 50µL of developer B. The well was gently shaked and mixed uniformly. The color developement was performed at 37°C for 15 minutes in the protection form light.
8. Termination: 50µL of a stop solution was added to each well to terminate the reaction (at this time, the blue color turned to the yellow color immediately).
9. Determination: The calibration to zero was performed according to the blank well, and then the absorbance (OD value) of each hole was measured in sequence at 450 nm wavelength. The determination should be carried out within 15 minutes after adding the stop solution.
10. Calculation: The concentration and OD value of the standard were used to calculate the linear regression equation of standard curve, y=bx+a, where x was the concentration and Y was the OD value. The OD value of the sample was substituted into the equation to calculate the sample concentration, which was then multiplied by the dilution factor to obtain the actual concentration of the sample.

The test results showed that Compound (I) (3 mpk, 10 mpk, 30 mpk and 60 mpk) and dexamethasone (3 mpk) significantly reduced the content of IL-6 in the skin (the groups of 3 mpk and 60 mpk of Compound (I): p<0.001 vs the model group; the groups of 10 mpk and 30 mpk of Compound (I) and the dexamethasone group: p<0.01 vs the model group) and the content of TNF-alpha in the skin (the group of 30 mpk of Compound (I): p<0.001 vs the model group; the group of 10 mpk of Compound (I): p<0.05 vs the model group; the groups of 3 mpk and 60 mpk of Compound (I) and the dexamethasone group: p<0.01 vs the model group). See Table 8 for details.

**Table 8: Skin inflammatory factor content of IMQ-induced mice after 14 days of treatment (mean±standard error)**

| Group | IL-6 (concentration pg/g) | TNF-alpha (concentration pg/g) |
|---|---|---|
| 1 | 544.08 ± 44.29*** | 906.85 ± 41.88*** |
| 2 | 1000.87 ± 72.20 | 1937.90 ± 129.72 |
| 3 | 554.74 ± 88.14*** | 1191.74 ± 124.81** |
| 4 | 641.83 ± 96.28** | 1432.02 ± 152.77* |
| 5 | 638.09 ± 59.63** | 1236.41 ± 100.8*** |
| 6 | 499.72 ± 95.81*** | 1188.81 ± 159.63** |
| 7 | 607.20 ± 76.89** | 1172.53 ± 115.72*** |

| | | |
|---|---|---|
| Note: **P*<0.05, ***P*<0.01, ****P*<0.001 vs G2 (model group) | | |

### Example 3: Pharmacodynamic test of Compound (I) in the OXA-induced mouse atopic dermatitis model

### 1. Test purpose

The purpose of this test was to evaluate the pharmacodynamics of Compound (I) in the OXA-induced Balb/c mouse atopic dermatitis model. Repeated OXA stimulation of the dorsal skin of mice could produce a long-term inflammatory reaction. This model was closer to the clinical skin inflammation and was a common model for screening and evaluating compounds with anti-inflammatory activity.

### 2. Test medicament

Tested medicament: Compound (I)
Positive control medicament: dexamethasone (Dex)
Vehicle (0.4% Tween 80/0.5% methyl cellulose).

### 3. Test animal

Female Balb/c mice aged 8-9 weeks, number: 70

### 4. Test grouping and dosage regimen

According to the body weight of animals in each group before administration, they were randomly divided into 7 groups with 10 animals in each group. See Table 9 for details.

**Table 9: Grouping of pharmacodynamic test of atopic dermatitis**

| Group | Aminal number | 5% OXA induction (Day -7) | Dose of Administration (mpk) | 0.1% OXA stimulation | Starting time of administration | Route and Frequency of Administration | Duration of Administration (day) |
|---|---|---|---|---|---|---|---|
| Normal control group | 10 | Acetone/olive oil (4/1) | / | Acetone/olive oil (4/1) | Day1 | PO bid | 22 |
| Model group | 10 | 5% OXA | / | 0.1% OXA | Day1 | PO bid | 22 |
| Compound (I) 3 mpk group | 10 | 5% OXA | 3 | 0.1% OXA | Day1 | PO bid | 22 |
| Compound (I) 10 mpk group | 10 | 5% OXA | 10 | 0.1% OXA | Day1 | PO bid | 22 |
| Compound (I) 30 mpk group | 10 | 5% OXA | 30 | 0.1% OXA | Day1 | PO bid | 22 |
| Compound (I) 60 mpk group | 10 | 5% OXA | 60 | 0.1% OXA | Day1 | PO bid | 22 |
| Dexamethasone 3 mpk group | 10 | 5% OXA | 3 | 0.1% OXA | Day1 | PO qd | 22 |

### Induction of atopic dermatitis model:

Mice were randomly divided into 7 groups according to the body weight, as shown in Table 9 above. OXA induction was performed on the 7th day. The induction mode was to apply 5% OXA (dissolved in the acetone/olive oil (4/1) solvent) to the back of mice near the neck in an amount of 10 µL (1.5 cm × 1.5 cm). Mice in the normal control group were applied with 10 µL of the acetone/olive oil (4/1) solvent in the same way. From the 1^{st} da to 22^{nd} day, the immunostimulation was performed, and 100 µL of 0.1% OXA (dissolved in the acetone/olive oil (4/1) solvent) was evenly applied on the back of mice near the neck. The application was performed every two days. Mice in the normal control group were applied with 100 µL of the acetone/olive oil (4/1) solvent in the same way. On the day of the immunostimulation, measuring the skin thickness, clinically scoring and photographing were required, and the OXA application was carried out after the above work was completed.

### Dosage regimen:

The administration dose of the compound was shown in Table 9. Compound (I) was administered by gavage twice a day (PO, bid) from the 1st to 22^{nd} day. The administration was performed in the morning and completed before measuring the skin thickness, clinically scoring, photographing, and the OXA immunostimulation. The interval between two administrations was 8 hours.

### 5. Test result

### 5.1 Influence of Compound (I) on the body weight of mice with the OXA-induced atopic dermatitis

Body weight data were recorded twice a week.

The test results showed that the OXA stimulation had no significant influence on the body weight of mice. At the end of the study, Compound (I) (3 mpk, 10 mpk, 30 mpk and 60 mpk) had no significant influence on the body weight of mice; and the body weight of mice decreased significantly after the dexamethasone treatment. See Table 10 for details.

**Table 10: Body weight of OXA-induced mice after 22 days of treatment (mean±standard error)**

| Group | Body weight (g) | | |
|---|---|---|---|
| | -7^{th} Day | 1^{st} Day | 22^{nd} Day |
| Normal control group | 18.56±0.17 | 18.56±0.17 | 19.65±0.28 |
| Model group | 18.12±0.13 | 18.12±0.13 | 19.37±0.18 |
| Compound (I) 3 mpk group | 18.11±0.13 | 18.11±0.13 | 18.83±0.20 |
| Compound (I) 10 mpk group | 18.11±0.13 | 18.11±0.13 | 18.80±0.22 |
| Compound (I) 30 mpk group | 18.10±0.12 | 18.10±0.12 | 18.79±0.11 |
| Compound (I) 60 mpk group | 18.10±0.12 | 18.10±0.12 | 18.92±0.13 |
| Dexamethasone 3 mpk group | 18.08±0.12 | 18.08±0.12 | 16.92***±0.20 |

| | | | |
|---|---|---|---|
| Note: **P*<0.05, ***P*<0.01, ****P*<0.001 vs the model group | | | |

### 5.2 Influence of Compound (I) on the skin thickness of mice with the OXA-induced atopic dermatitis

Skin thickness (1.5 cm×1.5 cm, the modeling area) was measured with a Mitutoyo digital indicator (Mitutoyo digimatic indicator, Type ID-C, USA) from the 1st to 22^{nd} day. The measurement was performed every two days. If the skin thickness measurement and the OXA immunostimulation were performed on the same day, the skin thickness measurement would be preferred.

The test results showed that after the OXA immunostimulation, the thickness of the dorsal skin of mice (1.5 cm×1.5 cm, the modeling area) significantly increased. From the 17^{th} day, compared with the model group, Compound(I) (30 mpk and 60 mpk) significantly inhibited the increase in the skin thickness of the modeling area (the group of 30 mpk on the 17^{th} and 22^{nd} days: p<0.05 vs the model group; the group of 60 mpk on the 17^{th} and 22^{nd} days: p<0.01 vs the model group). From the 7^{th} day, compared with the model group, dexamethasone significantly inhibited the increase in the skin thickness (the 7^{th} day: p<0.01 vs the model group; the 17^{th} and 22^{nd} days: p<0.001 vs the model group). See Table 11 for details.

**Table 11: Skin thickness of OXA-induced mice after 1-22 days of the treatment (mean±standard error, unit: mm)**

| Group | 1^{st} day | 7^{th} day | 17^{th} day | 22^{nd} day |
|---|---|---|---|---|
| Normal control group | 0.334***±0.007 | 0.356***±0.013 | 0.347***±0.013 | 0.376***±0.031 |
| Model group | 0.524±0.015 | 0.978±0.022 | 1.361±0.058 | 1.403±0.045 |
| Compound (I) 3 mpk group | 0.511±0.016 | 0.944±0.026 | 1.361±0.043 | 1.368±0.028 |
| Compound (I) 10 mpk group | 0.535±0.027 | 0.958±0.026 | 1.257±0.020 | 1.338±0.021 |
| Compound (I) 30 mpk group | 0.534±0.029 | 0.976±0.021 | 1.228*±0.063 | 1.268*±0.072 |
| Compound (I) 60 mpk group | 0.556±0.035 | 0.993±0.033 | 1.180**±0.054 | 1.238**±0.033 |
| Dexamethasone | 0.532±0.012 | 0.813**±0.023 | 0.646***±0.035 | 0.599***±0.025 |
| 3 mpk group | | | | |

| | | | | |
|---|---|---|---|---|
| Note: **P*<0.05, ***P*<0.01, ****P*<0.001 vs the model group. | | | | |

### 5.3 Skin clinical scoring of Compound (I) on the mice with the OXA-induced atopic dermatitis

The skin in the modeling area was clinically scored according to the skin scoring criteria in Table 12 from the 1st to 22^{nd} day and evaluated every two days. If the skin clinical scoring and the OXA immunostimulation were performed on the same day, the skin clinical scoring would be preferred.

**Table 12: Skin scoring criteria**

| Criteria | Score |
|---|---|
| Normal | 0 |
| Redness | 1 |
| Edema | 2 |
| Ecdysis | 3 |
| Effusion | 4 |

The test results showed that after the OXA immunostimulation, the skin clinical score in the modeling area of mice increased significantly. From the 19^{th} day, compared with the model group, Compound (I) (30 MPK and 60 MPK) significantly inhibited the increase of skin clinical score (both p<0.05 vs model group). From the 9^{th} day, compared with the model group, dexamethasone significantly inhibited the increase in the skin clinical score (the 9^{th} day: p<0.05 vs the model group; the 19^{th} and 22^{nd} days: p<0.001 vs the model group). See Table 13 for details.

**Table 13: Skin clinical score of the OXA-induced mice after 1-22 days of treatment (mean±standard error)**

| Group | 1^{st} Day | 9^{th} day | 19^{th} day | 22^{nd} day |
|---|---|---|---|---|
| Normal control group | 0.0±0.00 | 0.0***±0.00 | 0.0***±0.00 | 0.0***±0.00 |
| Model group | 0.4±0.16 | 3.2±0.13 | 3.0±0.00 | 3.0±0.00 |
| Compound (I) | 0.4±0.16 | 3.1±0.10 | 2.9±0.10 | 3.0±0.00 |
| 3 mpk group | | | | |
| Compound (I) | 0.4±0.16 | 3.0±0.00 | 2.7±0.15 | 2.7±0.15 |
| 10 mpk group | | | | |
| Compound (I) | 0.5±0.17 | 3.0±0.00 | 2.5*±0.17 | 2.5*±0.17 |
| 30 mpk group | | | | |
| Compound (I) | 0.6±0.16 | 3.0±0.00 | 2.5*±0.17 | 2.5*±0.17 |
| 60 mpk group | | | | |
| Dexamethasone | 0.4±0.16 | 2.7*±0.15 | 1.2***±0.13 | 1.1***±0.10 |
| 3 mpk group | | | | |

| | | | | |
|---|---|---|---|---|
| Note: **P*<0.05, ***P<0.01,* ****P*<0.001 vs the model group. | | | | |

### 5.4 Skin epidermal thickness measurement and pathological score

At the end of the study, the tissue in the modeling area was stained with hematoxylin eosin for the skin pathological scoring and the epidermal thickness measurement.

After the mice were sacrificed, the dorsal skin was removed and fixed in 10% neutral formalin for 24 hours. It was dehydrated, then embedded in paraffin and made into 4-micron sections. The skin sections were stained with hematoxylin eosin, and the inflammatory cell infiltration and tissue changes in the stratum corneum, the epidermis layer and the dermis layer were observed under the microscope, and the pathological scores were made. The scoring criteria were: inflammatory cell aggregation and edema, which were divided into slight 1 point, moderate 2 points, and severe 3 points; telangiectasia, which was divided into slight 1 point, moderate 2 points, and severe 3 points.

The specific scoring criteria of inflammatory cell aggregation were as follows: (1) slight-1 point: inflammatory cells occupied a small amount of <10% of the dermal area of the skin; (2) moderate-2 points: inflammatory cells occupied 10%-50% of the dermal area of the skin; (3) severe-3 points: the inflammatory cells occupied greater than 50% of the dermal area of the skin.

The specific scoring criteria of skin edema were as follows: (1) slight-1 point: there are occasionally several cell edema at the juncture between the dermis and the epidermis, and the edema cell aggregation length was less than 10% of the length of the juncture between the dermis and the epidermis; (2) moderate-2 points: the length of edema cells occupied 10%-50% of the length of the juncture between the dermis and the epidermis; (3) severe-3 points: the length of edema cells occupied greater than 50% of the length of the juncture between the dermis and the epidermis.

The specific scoring criteria of telangiectasia were as follows: (1) slight-1 point: occasionally 1-3 telangiectasias; (2) moderate-2 points: 3-6 telangiectasias; (3) severe-3 points: greater than 6 telangiectasias.

When measuring the thickness of the epidermal layer, the stained skin sections were scanned with a Leca Aperio CS2 scanner at 200 magnification, and then the scanned images were opened with HALO pathological analysis software. A "typing" template in the software was used to define the skin epidermis as an annotation layer, the epidermis was divided into upper and lower segments in the annotation layer, and then about 100 thickness values in the segments were calculated with the "layer thickness" option. The epidermal thickness of each section was represented by the average value of epidermal thickness. The test results showed that there were inflammatory cell aggregation, edema and telangiectasia in the skin of the modeling area of the model group. According to the pathological scoring criteria mentioned in the test method, the pathological score of this group reached 6.8. Compared with the model group, Compound (I) (10 mpk, 30 mpk and 60) could significantly reduce the pathological score of the skin in this area after treatment (p<0.01 vs the model group). After the dexamethasone treatment, the pathological score was significantly reduced (p<0.001 vs the model group). The OXA-stimulation significantly increased the thickness of the skin epidermal layer in the modeling area of the mouse back. Compared with the model group, Compound (I) (10 mpk, 30 mpk and 60 mpk) could significantly reduce the epidermal thickness (p<0.05 vs the model group); After the dexamethasone treatment, the thickness of the epidermal layer in this area decreased significantly (p<0.001 vs the model group). See Table 14 for details.

**Table 14: Pathological score and epidermal thickness of OXA-induced mice after 22 days of treatment**

| Group | Epidermal thickness (µm) | Pathological score |
|---|---|---|
| Normal control group | 4.34***±1.30 | 0.0***±0.00 |
| Model group | 86.76±3.55 | 6.8±0.33 |
| Compound (I) 3 mpk group | 75.79±3.36 | 6.4±0.34 |
| Compound (I) 10 mpk group | 68.84*±4.95 | 5.1**±0.43 |
| Compound (I) 30 mpk group | 75.08*±3.57 | 5.0**±0.42 |
| Compound (I) 60 mpk group | 72.90*±4.00 | 5.0**±0.52 |
| Dexamethasone 3 mpk group | 54.53***±2.98 | 1.1***±0.10 |

| | | |
|---|---|---|
| Note: **P*<0.05, ***P*<0.01, ****P*<0.001 vs the model group | | |

### Example 4: Pharmacodynamic test of Compound (I) on the SLE model mice

### 1. Test medicament

Tested medicament: Compound (I)
Positive control medicament: prednisone (SFDA Approval No. H33021207).

### 2. Test animal

female MRL/lpr mice aged 4 weeks, number: 50;
female C57BL/6 mice aged 4 weeks, number: 10.

### 3. Test grouping and dosage regimen

According to the concentration of the serum anti-ds-DNA antibody, female MRL/lpr mice were randomly divided into five groups: model group: solvent (aqueous solution containing 0.4% Tween 80 and 0.5% methylcellulose) 10 mL/kg BID, positive control group: prednisone 6 mg/kg QD, compound (I) 20, 40 and 60 mg/kg BID groups, the administration volumes respectively: 10 mL/kg. In addition, female C57BL/6 mice served as the normal control group. The animals in the Compound (I) group and the model group were administrated with the medicament or the solvent by gavage twice a day starting from the beginning of five weeks of age with an interval of 8 hours between two administrations; prednisone was administered to the animals by gavage once a day from the beginning of five weeks of age; the administration was totally performed for 17 weeks.

Two mice in the model group died at 15 and 16 weeks of administration, respectively, and 8 mice remained at the end of the test; one mouse in the compound (I) 20 mg/kg bid group died in the later period of the 16th week of administration, and 9 mice remained at the end of the test; and no death of mice occurred in other groups.

### 4. Test results

4.1 Influence of Compound (I) on the skin of the MRL/lpr mice
4.1.1 Skin destruction and score

The skin destructions on the face, ears and back of mice were observed and scored once a week for 17 times totally.

Scoring system: 1) red swelling and bleeding of the skin; 2) the extent of hair loss and skin dryness; 3) edema; 4) excoriation/corrosion; 5) lichenoid sclerotic plaque and other symptoms. The score of each item was as follows: normal =0 points; slight= 1 point; moderate = 2 points; severe =3 points. The severity of skin destruction was determined according to the total score of each evaluated symptom.

The results showed that compared with the model group, starting from the 14^{th} week of treatment, the high-dose and medium-dose groups of Compound (I) could effectively inhibit the skin destruction degree of systemic lupus erythematosus mice (the 14^{th}-16^{th} weeks, *p<0.05 vs the model group; the 17^{th} week, **p<0.01 vs the model group), and the positive control medicament prednisone 6 mg/kg group also effectively inhibited the skin destruction degree of the systemic lupus erythematosus mice (the 17^{th} week, **p<0.01 vs the model group). See Table 15 for details.

**Table 15: Skin scores of mice after 0-17 weeks of treatment**

| Week | Skin score, mean±standard error | | | | | |
|---|---|---|---|---|---|---|
| | Normal control group | Model group | Positive control group | Compound (I) group | Compound (I) group | Compound (I) group |
| | | | | 20 mg/kg | 40 mg/kg | 60 mg/kg |
| 0 | 0.0±0.00 | 0.0±0.00 | 0.0±0.00 | 0.0±0.00 | 0.0±0.00 | 0.0±0.00 |
| 4 | 0.0±0.00 | 0.0±0.00 | 0.0±0.00 | 0.0±0.00 | 0.0±0.00 | 0.0±0.00 |
| 8 | 0.0±0.00 | 0.0±0.00 | 0.3±0.15 | 0.0±0.00 | 0.0±0.00 | 0.0±0.00 |
| 12 | 0.0±0.00 | 0.1±0.10 | 0.3±0.15 | 0.0±0.00 | 0.0±0.00 | 0.0±0.00 |
| 14 | 0.0±0.00* | 0.3±0.21 | 0.2±0.13 | 0.2±0.20 | 0.0±0.00' | 0.0±0.00* |
| 16 | 0.0±0.00* | 0.3±0.24 | 0.1±0.10 | 0.2±0.13 | 0.0±0.00* | 0.0±0.00* |
| 17 | 0.0±0.00** | 0.4±0.24 | 0.1±0.10** | 0.2±0.15 | 0.0±0.00** | 0.0±0.00** |

| | | | | | | |
|---|---|---|---|---|---|---|
| Note: **P*<0.05, ***P*<0.01 vs the model group | | | | | | |

### 4.1.2 Skin HE pathological score

At the end of the test, the mice were dissected, the skin tissues on the back of mice were stained with HE and scored pathologically.

### Criteria for the skin HE pathological score

| Score | 0 | 1 | 2 | 3 | 4 |
|---|---|---|---|---|---|
| Hyperkeratosis/parakeratosis | No | slight | mild | moderate | severe |
| Horny follicular plug | No | slight | mild | moderate | severe |
| Atrophy of epidermis and dermis | No | slight | mild | moderate | severe |
| Superficial dermal edema | No | slight | mild | moderate | severe |
| Inflammatory cell infiltration | No | slight | mild | moderate | severe |
| Telangiectasia and hyperaemia | No | slight | mild | moderate | severe |
| Epidermis hyperplasia | No | slight | mild | moderate | severe |

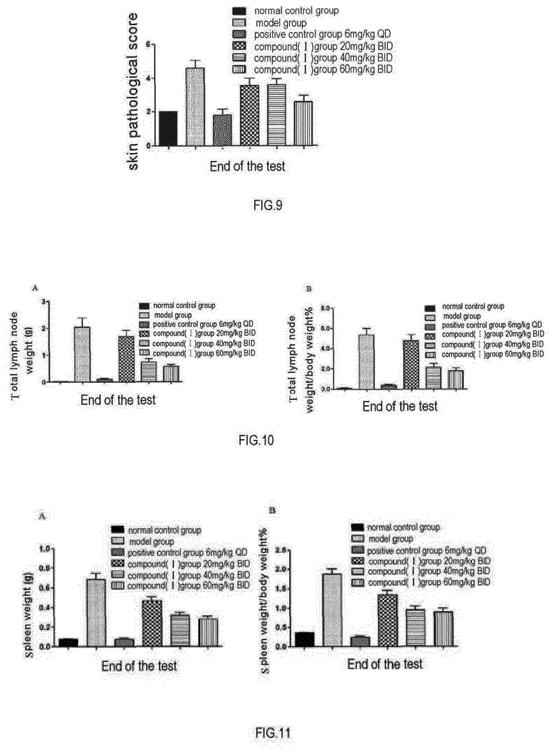

The results showed that each administration group could improve the skin pathology score to variours degrees. The high-dose group of Compound (I) groups showed a statistical difference (**p <0.01 vs the model group), and the positive control medicament prednisone 6 mg/kg group also showed a statistical difference (***p<0.001 vs the model group). See Table 16 and Figure 9 for the experimental results in details.

**Table 16: Skin HE pathological score of mice after 17 weeks of the treatment**

| | Skin HE pathological score, mean±standard error | | | | | |
|---|---|---|---|---|---|---|
| | Normal control group | Model group | Positive control group | Compound (I) group 20mg/kg | Compound (I) group 40mg/kg | Compound (I) group 60mg/kg |
| Score | 1.00±0.21*** | 4.63±0.46 | 1.80+0.36*** | 3.56±0.44 | 3.60±0.37 | 2.60±0.37** |

| | | | | | | |
|---|---|---|---|---|---|---|
| Note: ***P*<0.01, ****P*<0.001 vs the model group | | | | | | |

4.2 Influence of Compound (I) on the lymph node of MRL/lpr mice
4.2.1 Lymph node score

The lymph nodes of MRL/lpr mice were scored once a week for a total of 17 times during the test.

According to the lymph node diameter (cm), the score values were 0-6 points:
0 point: normal;
1 point: small (the diameter at one location of points on both sides was less than 1 cm);
2 points: small (the diameters at two location of points on both sides were less than 1 cm);
3 points: small (the diameters at three location of points on both sides were less than 1 cm);
4 points: large (the diameter at one location of points on both sides was larger than 1 cm, the diameters at the other two location of points on both sides were less than 1 cm);
5 points: large (the diameters at two location of points on both sides were larger than 1 cm, and the diameter at the other location of points on both sides was less than 1 cm);
6 points: large (the diameters at three location of points on both sides were greater than 1 cm).

According to the lymph node scoring data, after 7 weeks of administration and treatment, both high and medium doses of Compound (I) could effectively inhibit the lymphadenectasis degree in mice with systemic lupus erythematosus (***p<0.001 vs the model group), while the low-dose group could significantly inhibit the lymphadenectasis in the SLE mice after 7-11 weeks of administration (the 7^{th}-9^{th} weeks, ***p<0.001 vs the model group;; the 11^{th} week, *p<0.05 vs the model group), but the difference was no longer obvious from the 12^{th} week to the end of the experiment. The positive control medicament prednisone 6 mg/kg group also effectively inhibited the lymphadenectasis degree of mice with the systemic lupus erythematosus (***p <0.001 vs the model group). See Table 17 for details.

**Table 17: Lymph node score of mice after 17 weeks of treatment**

| Lymph node score, mean±standard error | | | | | | |
|---|---|---|---|---|---|---|
| Week | Normal control group | Model group | Positive control group | Compound (I) group | Compound (I) group | Compound (I) group |
| | | | | 20 mg/kg | 40 mg/kg | 60 mg/kg |
| 0 | 0.0±0.00 | 0.0±0.00 | 0.0±0.00 | 0.0±0.00 | 0.0±0.00 | 0.0±0.00 |
| 6 | 0.0±0.00 | 0.0±0.00 | 0.0±0.00 | 0.0±0.00 | 0.0±0.00 | 0.0±0.00 |
| 7 | 0.0±0.00*** | 1.6±0.22 | 0.2±0.13*** | 0.6±0.22*** | 0.3±0.15*** | 0.1±0.10*** |
| 9 | 0.0±0.00*** | 3.0±0.26 | 0.3±0.15*** | 2.0±0.26*** | 0.7±0.26*** | 0.3±0.15*** |
| 11 | 0.0±0.00*** | 3.5±0.27 | 0.0±0.00*** | 2.8±0.29* | 0.9±0.23 *** | 0.4±0.16 *** |
| 12 | 0.0±0.00*** | 3.7±0.21 | 0.0±0.00*** | 3.0±0.21 | 1.2±0.25*** | 0.5±0.22*** |
| 17 | 0.0±0.00*** | 4.1±0.23 | 0.0±0.00*** | 3.6±0.24 | 1.5±0.34*** | 1.0±0.26*** |

| | | | | | | |
|---|---|---|---|---|---|---|
| Note: **P*<0.05, ****P*<0.001 vs the model group | | | | | | |

### 4.2.2 Lymph node weight

At the end of the test, mice were dissected and the lymph node tissues (submaxillary, axillary and inguinal) were weighed. From the lymph node tissue weight data, compared with the model group, both high and medium doses of Compound (I) could effectively inhibit the lymphadenectasis degree of mice with systemic lupus erythematosus (***p <0.001 vs the model group). See Table 18 and Figure 10 for details.

**Table 18: Lymph node weight mean±standard error**

| | Normal control group | Model group | Positive control group | Compound (I) group | Compound (I) group | Compound (I) group |
|---|---|---|---|---|---|---|
| | | | | 20mg/kg | 40 mg/kg | 60 mg/kg |
| Total lymph node (g) | 0.0175± 0.00*** | 2.0416± 0.35 | 0.1123± 0.03*** | 1.6823± 0.24 | 0.7319± 0.12*** | 0.5680± 0.08"* |
| Total lymph node weight/bod y weight% | 0.0823± 0.01*** | 5.3365± 0.65 | 0.3445± 0.08*** | 4.7396± 0.60 | 2.1603± 0.35*** | 1.7879± 0.26*** |

| | | | | | | |
|---|---|---|---|---|---|---|
| Note: ****P*<0.001 vs the model group | | | | | | |

### 4.3 Influence of Compound (I) on spleen of MRL/lpr mice

At the end of the test, mice were dissected and the spleen tissues were weighed. Compared with the model group, all high, medium and low doses of Compound (I) could effectively inhibit the spleen enlargment degree of mice with systemic lupus erythematosus (***p <0.001 vs the model group). See Table 19 and Figure 11 for details.

**Table 19: Spleen weight and spleen weight/body weight (mean±standard error)**

| | Normal control group | Model group | Positive control group | Compound (I) group | Compound (I) group | Compound (I) group |
|---|---|---|---|---|---|---|
| | | | | 20 mg/kg | 40 mg/kg | 60 mg/kg |
| Spleen (g) | 0.0755± 0.00*** | 0.6799± 0.07 | 0.0756± 0.01*** | 0.4658± 0.04*** | 0.3184± 0.03"* | 0.2805± 0.03"* |
| Spleen weight/body weight% | 0.3556± 0.01*** | 1.8596± 0.14 | 0.2362± 0.03*** | 1.3336± 0.11*** | 0.9485± 0.09*** | 0.8892± 0.10*** |

| | | | | | | |
|---|---|---|---|---|---|---|
| Note: ****P*<0.001 vs the model group | | | | | | |

4.4 Influence of Compound (I) on the kidney of MRL/lpr mice
4.4.1 Area under the urinary protein curve

The urine protein contents of mice were detected during the test, once a week for 16 weeks. According to the urinary protein content at different time, the urinary protein concentration vs time curve was plotted and the area under each curve was calculated.

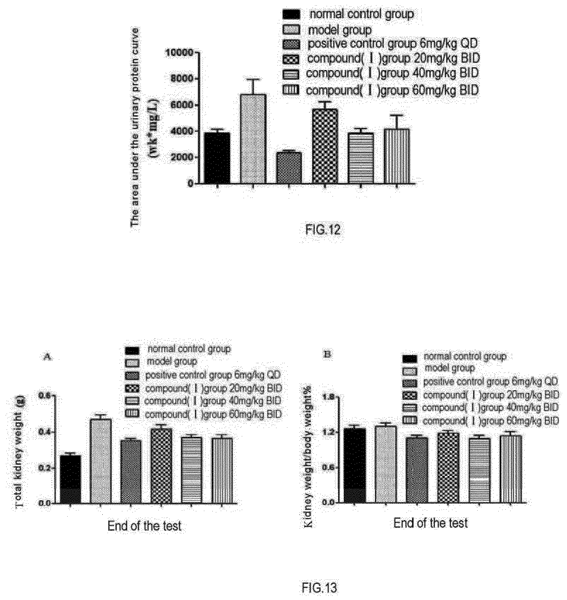

It could be seen that three dose groups of Compound (I) produced various degrees of improvement on the urinary protein, and the medium and high dose groups of Compound (I) had significant statistical difference in the area under the curve for the time of 16 weeks (*p <0.05 vs the model group). See Table 20 and Figure 12 for details.

**Table 20: Area under the urine protein curve of the mice after treatment for 16 weeks**

| | Area under the urine protein curve, mean±standard error, wk*mg/L | | | | | |
|---|---|---|---|---|---|---|
| | Normal control group | Model group | Positive control group | Compound (I) group 20 mg/kg | Compound (I) group 40 mg/kg | Compound (I) group 60 mg/kg |
| Area | 3856.14± 296.45* | 6809.65± 1140.71 | 2360.38± 161.30*** | 5646.42± 587.29 | 3851.50± 340.32* | 4138.70± 1048.57* |

| | | | | | | |
|---|---|---|---|---|---|---|
| Note: **P*<0.05, ****P*<0.001 vs the model group | | | | | | |

### 4.4.2 Kidney weight

At the end of the test, mice were dissected and the kidney tissues were weighed. Compared with the model group, the total pathological score of both kidneys in the high-dose group of Compound (I) was extremely significantly decreased (***p<0.001 vs the model group), and the total pathological score of both kidneys in the medium dose group of Compound (I) was significantly decreased (**p<0.01 vs the model group). See Table 21 and Figure 13 for details.

**Table 21: Total kidney weight and kidney weight/body weight of mice after 17 weeks of treatment**

| | Normal control group | Model group | Positive control group | Compound (I) group | Compound (I) group | Compound (I) group |
|---|---|---|---|---|---|---|
| | | | | 20 mg/kg | 40 mg/kg | 60 mg/kg |
| Total kidney | 0.2682± | 0.4685± | 0.3526± | 0.4146± | 0.3704± | 0.3639± |
| weight (g) | 0.01*** | 0.02 | 0.01*** | 0.02 | 0.01" | 0.02*** |
| Total kidney weight/body weight% | 1.2610± 0.05 | 1.2958± 0.07 | 1.1050± 0.04 | 1.1855± 0.05 | 1.0997± 0.05 | 1.1424± 0.07 |

| | | | | | | |
|---|---|---|---|---|---|---|
| Note: ***P*<0.01, ****P*<0.001 vs the model group | | | | | | |

### 4.4.3 Kidney HE pathological score

### Kidney HE scoring criteria:

### Activity index (AI) scoring criteria

| Pathological changes | Characteristics of pathological changes | | Point-value |
|---|---|---|---|
| Hyperplasia | Epithelial hyperplasia of the parietal layer invisible in the glomerular capsular space | (0) | 0 |
| | Slight epithelial hyperplasia of the parietal layer occasionally visible in the glomerular capsular space | (<5%) | 1 |
| | Epithelial hyperplasia of the parietal layer slightly visible in the glomerular capsular space | (5%∼25%) | 2 |
| | Epithelial hyperplasia of the parietal layer partially visible in the glomerular capsular space | (25%∼50%) | 3 |
| | Epithelial hyperplasia of the parietal layer widely visible in the glomerular capsular space | (>50%) | 4 |
| Cellular crescent | Crescent formation invisible in the glomerular capsular space | (0) | 0 |
| | Crescent formation occasionally visible in the glomerular capsular space | (<5%) | 1 |
| | Crescent formation slightly visible in the glomerular capsular space | (5%∼25%) | 2 |
| | Crescent formation partially visible in the glomerular capsular space | (25%∼50%) | 3 |
| | Crescent formation widely visible in the | (>50%) | 4 |
| | glomerular capsular space | | |
| Neutrophilic granulocyte infiltration | Neutrophilic granulocyte infiltration invisible in the glomeruli | (0) | 0 |
| | Neutrophilic granulocyte infiltration ocassionally visible in the glomeruli | (<5%) | 1 |
| | Neutrophilic granulocyte infiltration slightly visible in the glomeruli | (5%∼25%) | 2 |
| | Neutrophilic granulocyte infiltration partially visible in the glomeruli | (25%∼50%) | 3 |
| | Neutrophilic granulocyte infiltration widely visible in the glomeruli | (>50%) | 4 |
| Infiltration of inflammatory cells around glomeruli | Infiltration of inflammatory cells invisible around glomeruli | (0) | 0 |
| | Infiltration of inflammatory cells ocassionally visible around glomeruli | (<5%) | 1 |
| | Infiltration of inflammatory cells slightly visible around glomeruli | (5%∼25%) | 2 |
| | Infiltration of inflammatory cells partially visible around glomeruli | (25%∼50%) | 3 |
| | Infiltration of inflammatory cells widely visible around glomeruli | (>50%) | 4 |
| Fibrinoid necrosis | Neither the coagulative compact eosinophilic substance appeared nor the fibrinoid necrosis formed in glomeruli | (0) | 0 |
| | The coagulative compact eosinophilic substance ocassionally appeared, and the fibrinoid necrosis locally and occasionally formed in glomeruli | (<5%) | 1 |
| | The coagulative compact eosinophilic substance slightly appeared, and the fibrinoid necrosis slightly formed in glomeruli | (5%∼25%) | 2 |
| | The coagulative compact eosinophilic | (25%∼50%) | 3 |
| | substance partially appeared, and the fibrinoid necrosis partially formed in glomeruli | | |
| | The coagulative compact eosinophilic substance widely appeared, and the fibrinoid necrosis widely formed in glomeruli | (>50%) | 4 |
| Glomerulonephritis activity index (AI) was the sum of the above five scores | | | 0-20 |

### Chronicity index (CI) scoring criteria

| Pathological changes | Degree of pathological changes | Point-value |
|---|---|---|
| Glomerular sclerosis (segmental or global) | No | 0 |
| | Mild | 1 |
| | Moderate | 2 |
| | Severe | 3 |
| Fibrous crescent | No | 0 |
| | Mild | 1 |
| | Moderate | 2 |
| | Severe | 3 |
| Interstitial fibrosis | No | 0 |
| | Mild | 1 |
| | Moderate | 2 |
| | Severe | 3 |
| Renal tubular atrophy | No | 0 |
| | Mild | 1 |
| | Moderate | 2 |
| | Severe | 3 |
| Chronicity index (CI) was the sum of the above four scores | | 0-12 |

### Tubulointerstitial lesions (TIL) scoring criteria

| Pathological changes | Degree of pathological changes | Point-value |
|---|---|---|
| Renal tubular degeneration and necrosis | No | 0 |
| | Mild | 1 |
| | Moderate | 2 |
| | Severe | 3 |
| Renal tubular atrophy | No | 0 |
| | Mild | 1 |
| | Moderate | 2 |
| | Severe | 3 |
| Interstitial inflammatory cell infiltration | No | 0 |
| | Mild | 1 |
| | Moderate | 2 |
| | Severe | 3 |
| Interstitial fibrosis | No | 0 |
| | Mild | 1 |
| | Moderate | 2 |
| | Severe | 3 |
| Renal tubule interstitial injury (TIL) was the sum of the above four scores | | 0-12 |

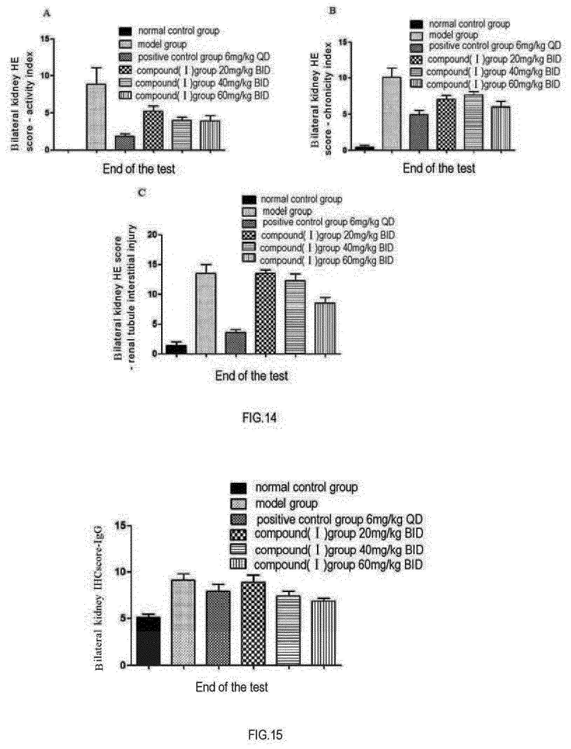

HE staining score results: compared with the model group, in the activity index (AI) score, the total pathological scores of both kidneys in the medium and high dose groups of Compound (I) were significantly decreased (**p<0.01 vs the model group); in the chronicity index (CI) score, the total pathological score of both kidneys in the high dose group of Compound (I) was significantly decreased (**p<0.01 vs the model group), the total pathological score of both kidneys in the low dose group of Compound (I) was relatively remarkably decreased (*p<0.05 vs the model group); in the tubulointerstitial lesions (TIL) score, the total pathological score of both kidneys in the high dose group of Compound (I) was significantly decreased (**p<0.01 vs the model group). See Table 22 and Figure 14 for details.

**Table 22: Kidney HE pathological score of mice after 17 weeks of treatment**

| Score | Pathological score of both kidneys, mean±standard error | | | | | |
|---|---|---|---|---|---|---|
| | Normal control group | Model group | Positive control group | Compound (I) group 20 mg/kg | Compound (I) group 40 mg/kg | Compound (I) group 60 mg/kg |
| Activity index | 0.00±0.00*** | 8.80±2.28 | 1.80+0.36*** | 5.20±0.73 | 4.00±0.42** | 3.90±0.75** |
| Chronicity index | 0.40±0.22*** | 10.10±1.31 | 4.90±0.62*** | 7.00±0.54* | 7.60±0.50 | 6.00±0.75** |
| Tubulointerstitial lesions | 1.40+0.56*** | 13.50±1.50 | 3.60±0.45*** | 13.50±0.52 | 12.30±1.03 | 8.50±0.91** |

| | | | | | | |
|---|---|---|---|---|---|---|
| Note: **P*<0.05, ***P*<0.01, ****P*<0.001 vs the model group | | | | | | |

The positive cell rate of IgG staining and the staining intensity were scored by IHC staining of kidney tissue.

### Kidney IHC (IgG) scoring criteria:

### IHC scoring criterion I

| Staining intensity | Score value |
|---|---|
| negative | 0 |
| weak | 1 |
| moderate | 2 |
| strong | 3 |

### IHC scoring criterion II

| Cell positive rate | Score value |
|---|---|
| No positive cells | 0 |
| Cell positive rate≤10% | 1 |
| 10%<Cell positive rate≤50% | 2 |
| 50%<Cell positive rate≤80% | 3 |
| Cell positive rate>80% | 4 |

The final scoring result was the product of the two, and 0 is negative (-); 1-3 were classified as low expression (+); 4-8 were classified as medium expression (+ +); 9-12 were classified as high expression (+ + +).

The results showed that compared with the model group, each dose group of Compound (I) improved IgG deposition in kidney tissue to various degrees, and the total IHC staining pathological score of both kidneys in the high-dose group of Compound (I) was significantly decreased (*p<0.05 vs the model group). See Table 23 and Figure 15 for details.

**Table 23: Kidney IHC pathological score of mice after 17 weeks of treatment**

| Score | Kidney IHC pathological score, mean±standard error | | | | | |
|---|---|---|---|---|---|---|
| | Normal control group | Model group | Positive control group | Compound (I) group 20 mg/kg | Compound (I) group 40 mg/kg | Compound (I) group 60 mg/kg |
| Bilateral kidneys | 5.10±0.35*** | 9.10±0.71 | 7.90±0.77 | 8.90±0.72 | 7.40±0.56 | 6.90±0.28* |

| | | | | | | |
|---|---|---|---|---|---|---|
| Note: **P*<0.05, ****P*<0.001 vs the model group | | | | | | |

### 4.5 Influence of Compound (I) on the concentration of serum anti-ds-DNA antibody of MRL/lpr mice

During the test, the concentration of serum anti-ds-DNA antibody of mice were detected every 4 weeks for 16 weeks.

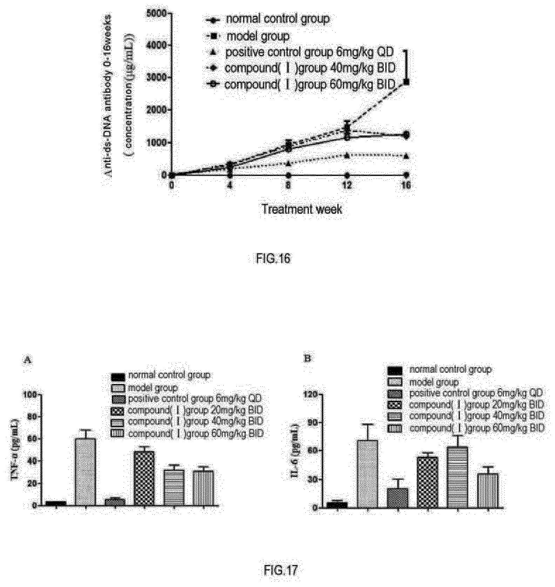

The results showed that the symptoms of lupus erythematosus of mice in the model group were aggravated with the test time. It could be seen from the concentration of anti-ds-DNA antibody that, after 3-4 weeks of treatment, compared with the model group, the medium dose group of Compound (I) could effectively reduce the concentration level of anti-ds-DNA antibody (16 weeks, *p<0.05 vs the model group). The positive control medicament prednisone 6 mg/kg could also effectively reduce the concentration level of anti-ds-DNA antibody. See Table 24 and Figure 16 for details.

**Table 24: Concentration of anti-ds-DNA antibody of mice after 0-16 weeks of treatment**

| Week | Concentration of anti-ds-DNA antibody (expressed in µg/mL), mean±standard error | | | | |
|---|---|---|---|---|---|
| | Normal control group | Model group | Positive control group | Compound (1) group 40 mg/kg | Compound (1) group 60 mg/kg |
| 0 | 1.83+0.01 | 3.30±0.39 | 3.26±0.30 | 3.32±0.39 | 3.33±0.42 |
| 4 | 2.13±0.03 | 319.71±25.79 | 183.55+28.07 | 346.06±35.04 | 249.26±24.12 |
| 8 | 2.60±0.16 | 943.95±131.31 | 363.75±18.45 | 885.04±96.38 | 806.75±110.22 |
| 12 | 3.01±0.30 | 1475.64±182.77 | 619.78+25.47 | 1390.83±266.11 | 1168.19±113.75 |
| 16 | 4.71±0.73*** | 2884.10±952.05 | 606.66±26.65** | 1198.59±86.64* | 1272.27±50.19 |

| | | | | | |
|---|---|---|---|---|---|
| Note: **P*<0.05, ***P*<0.01, ****P*<0.001 vs the model group | | | | | |

### 4.6 Influence of Compound (I) on the serum cytokines of MRL/lpr mice

At the end of the test, the serum cytokine levels of mice were detected by ELISA. The results showed that compared with the model group, low, medium and high doses, three dose groups of Compound (I) all reduced the concentration level of TNF-α, and there were significant differences between the medium/high dose groups and the model group (***p<0.001 vs the model group). Three dose groups of Compound (I) also had a certain reduction effect on IL-6 factor, and the high dose group had a significant effect on the IL-6 reduction. See Table 25 and Figure 17 for details.

**Table 25: Cytokine concentrations in serum at the end of the test**

| Cytokines | Mean ± standard error, concentration (pg/mL) | | | | | |
|---|---|---|---|---|---|---|
| | Normal control group | Model group | Positive control group | Compound (I) group 20 mg/kg | Compound (I) group 40 mg/kg | Compound (I) group 60 mg/kg |
| IL-6 | 5.19±2.61*** | 70.78±17.46 | 19.91±10.43** | 52.38±5.29 | 63.17±12.60 | 35.99±7.36 |
| TNF-alpha | 3.22±0.32*** | 59.65±7.99 | 5.50±1.13*** | 48.03±4.12 | 32.29±4.62*** | 30.61±4.72*** |

| | | | | | | |
|---|---|---|---|---|---|---|
| Note: ***P*<0.01, ****P*<0.001 vs the model group | | | | | | |

### Example 5: Toxicology experiment

According to the general toxicology study in accordance with the NMPA "Good Laboratory Practice for Non-clinical Laboratory Studies" and FDA GLP specification (21CFR Part 58), and in line with the requirements of International Conference on Harmonization of Technical Requirements for Registration of Pharmaceuticals for Human Use (ICH) and the NMPA relevant guidelines, the toxicology study on Compound (I) was carried out to provide safety data support for the use of Compound (I) in clinical patients. The administration mode and the experimental results were as follows:

| Experiment name | Dose and mode of administration |
|---|---|
| Toxicity test of single oral gavage administration in SD rats | 0 (Vehicle), 300, 800 and 2000 mg/kg |
| Toxicity test of single oral gavage administration in Beagle dogs | 0 (vehicle), 125, 250 and 500 mg/kg/time, administered twice within 24 hours with an interval of at least 4 hours |
| Toxicity test of oral gavage administration in SD rats with four-week recovery and four-week repetition | Vehicle or 10, 30, and 100 mg/kg/time, orally administered to SD rats twice a day with an interval of more than 4 hours for 28 consecutive days |
| Toxicity test of oral gavage administration in Beagle dogs with four-week recovery and four-week repetition | Vehicle or 3, 10, and 30 mg/kg/time, orally administered to Beagle dogs twice a day with an interval of more than 4 hours for 28 consecutive days |

### Experimental results:

Compound (I) had no effect on the central nervous system, respiratory system and cardiovascular system of animals, and was expected to have no side effects on the central nervous system, respiratory system and cardiovascular system of human. The NOAEL of single administration to rats was 2000 mg/kg, the MTD of single administration to Beagle dogs was 500 mg/kg/time (1000 mg/kg/day), the NOAEL of repeated administration to rats for 28 days was 10 mg/kg/time (20 mg/kg/day), and the NOAEL of repeated administration to Beagle dogs for 28 days was 3 mg/kg/time (6 mg/kg/ day). Compound (I) had no genotoxicity. The safety window assessment was shown in the table below. The effective dose for rats was 3 mg/kg/time bid. According to the exposure, Compound (I) had 1-fold safety window in Beagles and 8-13-fold safety window in SD rats. According to the dose calculation, Compound (I) had 3-fold safety windows in both Beagle dogs and SD rats. The proposed and undetermined initial ascending dose of Compound (I) in the first-in-human trial was 15 mg. Compound (I) had a 14-fold safety window for the human equivalent dose of the NOAEL dose in the Beagle dog repeated administration toxicology study relative to the human initial dose, and a 16-fold safety window for the human equivalent dose of the NOAEL dose in the SD rat repeated administration toxicology study relative to the human initial dose. Therefore, according to the pre-clinical toxicological study data of Compound (I), the single ascending doses of phase I clinical trial to be carried out were 15 mg, 45 mg, 60 mg, 90 mg, 135 mg, 180 mg, 240 mg, 300 mg and 360 mg.

**Table 26: Calculation of safety window of Compound (I)**

| Experimental project | Animal dose (mg/kg/time BID) | AUC (h*ng/mL) | Human equivalent dose (mg, based on 60 kg body weight) | Safety window (fold) | |
|---|---|---|---|---|---|
| | | | | Dose method | Exposure method |
| Rat effective dose | 3 | 4310 | 73 | / | / |
| NOAEL for 28-day test | 10 | ♂33900 | 242 | 3 | ♂8 |
| of rats | | ♀58100 | | | ♀13 |
| NOAEL for 28-day test of dogs | 3 | ♂3300 | 210 | 3 | 1 |
| | | ♀3930 | | | |

The results of phase I clinical trial showed that when the single ascending dose came up to 240 mg, there were no safety issues above Level 3.

In conclusion, Compound (I) could improve the skin lesions of the psoriasis mice and the atopic dermatitis mice, inhibit the immune organ enlargement, and reduce the level of inflammation; it could also dose-dependently improve the skin lesions of the SLE mice, alleviate the kidney injury, inhibit the immune organ enlargement, and inhibit the increase of SLE related antibodies and cytokines in serum, and has a certain safe therapeutic window, resulting in a good clinical application prospect.

The full names for the English abbreviations used in this application and the corresponding Chinese names are as follows:

| Abbreviations | Names in English | Names in Chinese |
|---|---|---|
| IL | Interleukin | 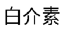 |
| IFN | Interferon | 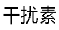 |
| EPO | Erythropoietin | 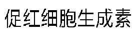 |
| Dectin-1 | Dendritic cell-associated C-type lectin-1 | 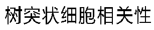 C 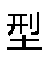 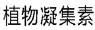 |
| ITAM | Immunoreceptor tyrosine activation motif | 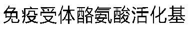 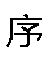 |
| BCR | B cell receptor | B 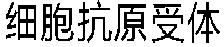 |
| CARD9 | Caspase recruitment domain 9 | 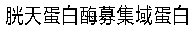 9 |
| NF-κB | Nuclear factor κB | 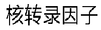 κB |
| Caspase-8 | Cysteinyl aspartate specific protease 8 | 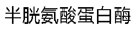 8 |
| NLRP3 | NLR Family, Pyrin Domain Containing Protein 3 (NLRP3) | NLRP3 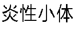 |
| Th | helper T cell | 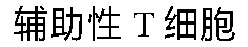 |
| HE | Hematoxylin-Eosin stain | 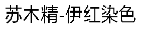 |
| IMQ | Imiquimod | 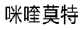 |
| MC | Methylcellulose | 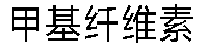 |
| PASI | Psoriasis area and severity index | 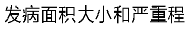 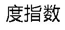 |
| ELISA | Enzyme linked immunosorbent assay | 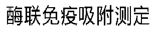 |
| OXA | 4-Ethoxymethylene-2-phenyl-2-oxazolin-5-one | 4- 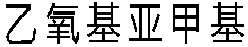 -2- 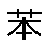 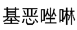 -5- 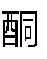 |
| Dex | Dexamethasone | 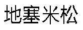 |
| NOAEL | No observed adverse effect level | 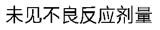 |

## Claims

1. Use of Compound (I), an optical isomer thereof or a pharmaceutically acceptable salt thereof in manufacture of a medicament for treating a disease related to high expression or abnormal activation of JAK and SYK kinases, wherein the disease related to high expression or abnormal activation of JAK and SYK kinases is an autoimmune disease, and the structure of said Compound (I) is as shown in the following formula:

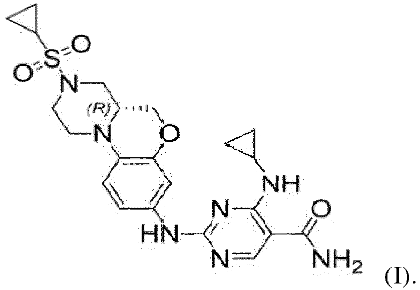

2. The use according to claim 1, wherein the autoimmune disease is selected from a group consisting of an immune-mediated skin disease and an autoimmune connective tissue disease.

3. The use according to claim 2, wherein the immune-mediated skin disease is psoriasis or atopic dermatitis, the psoriasis is preferably psoriasis vulgaris, pustular psoriasis, erythroderma psoriaticum or psoriasis arthropathica; wherein the autoimmune connective tissue disease is lupus erythematosus, the lupus erythematosus is preferably discoid lupus erythematosus, subacute cutaneous lupus erythematosus, systemic lupus erythematosus, lupus erythematosus profundus, neonatal lupus erythematosus, medicament-induced lupus erythematosus, further preferably systemic lupus erythematosus.

4. The use according to any one of claims 1-3, which is **characterized in that** the medicament contains a therapeutically effective amount of Compound (I), an optical isomer thereof or a pharmaceutically acceptable salt thereof, and optionally a pharmaceutically acceptable excipient or carrier.

5. The use according to claim 4, which is **characterized in that** the medicament is made into clinically acceptable dosage forms, including oral dosage forms, injection dosage forms, topical dosage forms or external dosage forms.

6. The use according to any of claims 1-3, which is **characterized in that** the medicament is clinically used alone or in combination with other therapeutic component(s).

7. A medicament comprising a therapeutically effective amount of Compound (I), an optical isomer thereof or a pharmaceutically acceptable salt thereof, 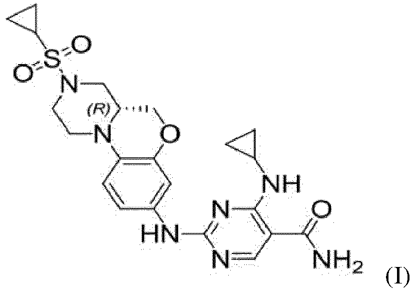 wherein the medicament is useful for treating a disease related to high expression or abnormal activation of JAK and SYK kinases in a subject, wherein said disease is an autoimmune disease, such as an immune-mediated skin disease and an autoimmune connective tissue disease, the immune-mediated skin disease is psoriasis or atopic dermatitis, the psoriasis is preferably psoriasis vulgaris, pustular psoriasis, erythroderma psoriaticum or psoriasis arthropathica; the autoimmune connective tissue disease is lupus erythematosus, the lupus erythematosus is preferably discoid lupus erythematosus, subacute cutaneous lupus erythematosus, systemic lupus erythematosus, lupus erythematosus profundus, neonatal lupus erythematosus, medicament-induced lupus erythematosus, further preferably systemic lupus erythematosus.

8. A method for treating a disease related to high expression or abnormal activation of JAK and SYK kinases, comprising administration of a therapeutically effective amount of Compound (I), an optical isomer thereof or a pharmaceutically acceptable salt thereof to a mammal (such as human) in need thereof: 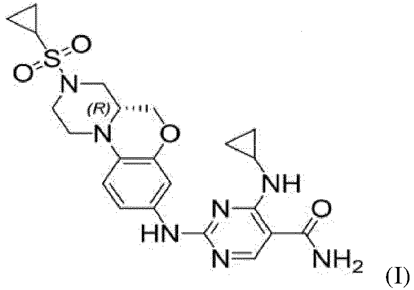 the disease related to high expression or abnormal activation of JAK and SYK kinases is an autoimmune disease, such as an immune-mediated skin disease and an autoimmune connective tissue disease, the immune-mediated skin disease is psoriasis or atopic dermatitis, the psoriasis is preferably psoriasis vulgaris, pustular psoriasis, erythroderma psoriaticum or psoriasis arthropathica; the autoimmune connective tissue disease is lupus erythematosus, the lupus erythematosus is preferably discoid lupus erythematosus, subacute cutaneous lupus erythematosus, systemic lupus erythematosus, lupus erythematosus profundus, neonatal lupus erythematosus, medicament-induced lupus erythematosus, further preferably systemic lupus erythematosus.

9. A compound medicament or combination product, wherein the compound medicament or combination product contains a therapeutically effective amount of Compound (I), an optical isomer thereof or a pharmaceutically acceptable salt thereof, and other therapeutic component(s), 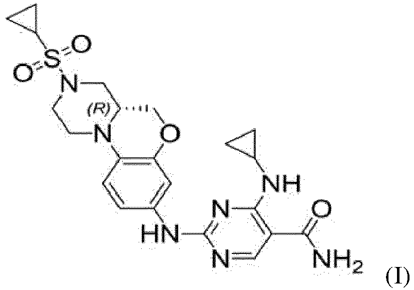 which is **characterized in that** the compound medicament or combination product is useful for treating a disease related to high expression or abnormal activation of JAK and SYK kinases in a subject, wherein said disease is an autoimmune disease, such as an immune-mediated skin disease and an autoimmune connective tissue disease, the immune-mediated skin disease is psoriasis or atopic dermatitis, the psoriasis is preferably psoriasis vulgaris, pustular psoriasis, erythroderma psoriaticum or psoriasis arthropathica; the autoimmune connective tissue disease is lupus erythematosus, the lupus erythematosus is preferably discoid lupus erythematosus, subacute cutaneous lupus erythematosus, systemic lupus erythematosus, lupus erythematosus profundus, neonatal lupus erythematosus, medicament-induced lupus erythematosus, further preferably systemic lupus erythematosus.

10. The use according to claim 4, which is **characterized in that** the therapeutic effective amount is 0.01-2000 mg, preferably 1-500 mg, more preferably 10-400 mg, further preferably 15-360 mg or 15-250 mg, such as 15 mg, 45 mg, 90 mg, 135 mg, 180 mg, 240 mg, 300 mg, 360 mg.
